(19) Europäisches Patentamt
European Patent Office
Office européen des brevets

(11) **EP 4 053 094 A1**

(12) **EUROPEAN PATENT APPLICATION**
published in accordance with Art. 153(4) EPC

(43) Date of publication:
**07.09.2022 Bulletin 2022/36**

(21) Application number: **20882151.2**

(22) Date of filing: **14.10.2020**

(51) International Patent Classification (IPC):
***C07C 22/08*** *(2006.01)* ***C07C 25/22*** *(2006.01)*
***C07C 255/51*** *(2006.01)* ***C07C 255/52*** *(2006.01)*
***C07C 255/54*** *(2006.01)* ***C09K 11/06*** *(2006.01)*
***G02B 5/20*** *(2006.01)*

(52) Cooperative Patent Classification (CPC):
**C07C 22/08; C07C 25/22; C07C 255/51; C07C 255/52; C07C 255/54; C09K 11/06; G02B 5/20**

(86) International application number:
**PCT/JP2020/038736**

(87) International publication number:
**WO 2021/085131 (06.05.2021 Gazette 2021/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.11.2019 JP 2019200318**

(71) Applicant: **CANON KABUSHIKI KAISHA**
**OHTA-KU**
**Tokyo 146-8501 (JP)**

(72) Inventors:
- **SHIOBARA Satoru**
  **Tokyo 146-8501 (JP)**
- **YAMADA Naoki**
  **Tokyo 146-8501 (JP)**
- **MIYASHITA Hirokazu**
  **Tokyo 146-8501 (JP)**
- **ITO Yuto**
  **Tokyo 146-8501 (JP)**
- **KAWATA Isao**
  **Tokyo 146-8501 (JP)**
- **KAMATANI Jun**
  **Tokyo 146-8501 (JP)**

(74) Representative: **TBK**
**Bavariaring 4-6**
**80336 München (DE)**

(54) **ORGANIC COMPOUND AND ORGANIC LIGHT-EMITTING ELEMENT**

(57) The present disclosure provides an organic compound that has a mother skeleton with a fused-ring structure, an electron-withdrawing group bonded to the mother skeleton, and an electron-donating group bonded to the mother skeleton, wherein the electron-withdrawing group is bonded at a position satisfying the following relationship in the mother skeleton.

$$\Sigma|C_H| > \Sigma|C_L| \quad (1)$$ (1)

($C_H$: 2PZ atomic orbital coefficient of a carbon at a substitution site in the HOMO of the mother skeleton)
($C_L$: 2PZ atomic orbital coefficient of the carbon at the substitution site in the LUMO of the mother skeleton)

FIG. 1C

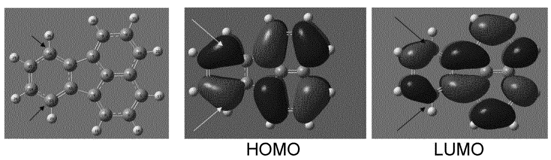

HOMO LUMO

EP 4 053 094 A1

## Description

Technical Field

The present invention relates to an organic compound and an organic light-emitting element including the organic compound.

Background Art

In recent years, self-luminous devices for flat panels have attracted attention. Examples of the self-luminous devices include plasma emission display elements, field emission elements, and organic light-emitting elements. Among these, in particular, organic light-emitting elements have been actively researched and developed. In particular, expanding the color reproduction range of displays is one technical issue, and attempts are continued to expand the color reproduction range by developing a device structure of an organic light-emitting element and developing a light-emitting material. sRGB and Adobe RGB standards are used for a color reproduction range for displays, and materials for reproducing these standards have been studied. BT-2020 has recently been proposed as a standard to further expand the color reproduction range.

It is known that the color reproduction range of an organic light-emitting element is expanded by improving the color purity of a light-emitting material, and various light-emitting materials have been developed.

Patent Literature 1 describes light-emitting materials with various substituents.

Citation List

Patent Literature

PTL 1: Japanese Patent Laid-Open No. 11-40360

Summary of Invention

Technical Problem

It is known that a substituent introduced into a molecular structure of an organic compound increases the emission wavelength of the organic compound. This is due to the conjugation length expansion effect and broken molecular symmetry. Thus, it has been considered difficult to emit light at a wavelength shorter than the wavelength of an organic compound by introducing a substituent into the organic compound. Thus, it has been believed that the molecular structure of a light-emitting material must be redesigned from the beginning, for example, by changing the basic skeleton of the molecular structure, to produce an organic compound that emits light with a shorter wavelength. However, it is not easy to redesign the molecular structure to develop a light-emitting material with high color purity, and there has been a demand for a light-emitting material with high color purity based on an existing skeleton. Solution to Problem

In view of these problems, it is an object of the present invention to provide a light-emitting material with high color purity.

An embodiment of the present invention provides an organic compound that has a mother skeleton with a fused-ring structure, an electron-donating group bonded to the mother skeleton, and an electron-withdrawing group bonded to the mother skeleton, wherein the electron-withdrawing group is bonded at a position satisfying the following relationship in the mother skeleton.

$$\Sigma|C_H| > \Sigma|C_L| \qquad (1)$$

($C_H$: 2PZ atomic orbital coefficient of a carbon at a substitution site in the HOMO of the mother skeleton)
($C_L$: 2PZ atomic orbital coefficient of the carbon at the substitution site in the LUMO of the mother skeleton) Advantageous Effects of Invention

The present invention can provide an organic compound with a shorter emission wavelength and high color purity due to an electron-withdrawing group and an electron-donating group.

Brief Description of Drawings

[Fig. 1A] Fig. 1A is an explanatory view of the mechanism of the effect of shortening the wavelength by substitution of an electron-withdrawing group.

[Fig. 1B] Fig. 1B is an explanatory view of the mechanism of shortening the wavelength by substitution of an electron-donating group.

[Fig. 1C] Fig. 1C is an explanatory view of the characteristics of the substitution position of an electron-withdrawing group.

[Fig. 1D] Fig. 1D is a diagram of an example of a mother skeleton without the orbital coefficient of LUMO.

[Fig. 1E] Fig. 1E is a diagram of chemical bonds and their binding energies.

[Fig. 2A] Fig. 2A is a schematic cross-sectional view of an example of a display apparatus including an organic light-emitting element according to an embodiment of the present invention.

[Fig. 2B] Fig. 2B is a schematic cross-sectional view of an example of a display apparatus including an organic light-emitting element according to another embodiment of the present invention.

[Fig. 3] Fig. 3 is a schematic view of an example of a display apparatus according to an embodiment of the present invention.

[Fig. 4A] Fig. 4A is a schematic view of an example of an imaging apparatus according to an embodiment of the present invention.

[Fig. 4B] Fig. 4B is a schematic view of an example of electronic equipment according to an embodiment of the present invention.

[Fig. 5A] Fig. 5A is a schematic view of an example of a display apparatus according to an embodiment of the present invention.

[Fig. 5B] Fig. 5B is a schematic view of an example of a foldable display apparatus.

[Fig. 6A] Fig. 6A is a schematic view of an example of a lighting apparatus according to an embodiment of the present invention.

[Fig. 6B] Fig. 6B is a schematic view of an example of an automobile with a vehicle lamp according to an embodiment of the present invention.

Description of Embodiments

An organic compound according to an embodiment of the present invention has the following structure and can therefore shorten the emission wavelength. This can improve the chromaticity coordinates and expand the color reproduction range in an organic light-emitting element. Furthermore, the organic compound according to the present embodiment can be used to provide an organic light-emitting element with high durability. The term "mother skeleton", as used herein, refers to a chemical structure represented by a fused-ring structure alone. For example, in a structure represented by a general formula, a structural formula in which all substituents are hydrogen atoms is a mother skeleton.

In one embodiment of the present invention, an electron-withdrawing group (A) and an electron-donating group (D) introduced into a mother skeleton (M) act on the highest occupied molecular orbital (HOMO) and lowest unoccupied molecular orbital (LUMO) and shorten the emission wavelength of the organic compound. The mother skeleton may have a tricyclic or higher polycyclic fused-ring structure.

(1) Shorter Wavelength due to Electron-Withdrawing Group

Figs. 1A to 1E are diagrams of one example of a molecular orbital of an organic compound. Fig. 1A illustrates M(HOMO) and M(LUMO), which represent the energy of a mother skeleton alone. On the other hand, M-A(HOMO) and M-A(LUMO) represent the energy of an organic compound with a mother skeleton into which an electron-withdrawing group is introduced. Introduction of the electron-withdrawing group lowers the energy of HOMO and LUMO. The lower energy position is the lower side on the drawing in Fig. 1A.

In this case, a shift in HOMO greater than a shift in LUMO increases the energy difference between the HOMO and the LUMO, that is, the band gap, and shortens the emission wavelength of the organic compound. This is because the organic compound emits light with a wavelength corresponding to the band gap energy.

To introduce an electron-withdrawing group to shorten the emission wavelength, the electron-withdrawing group is preferably introduced at a substitution position in the mother skeleton at which the substituent is more effective for the HOMO than the LUMO. More specifically, the orbital coefficient of the HOMO ($C_H$) of the carbon atom substituted by the electron-withdrawing group is preferably larger than the orbital coefficient of the LUMO ($C_L$). The orbital coefficient is a coefficient representing the degree of interaction with a substituent. A larger orbital coefficient results in greater

interaction with a substituent and a greater change in energy level. More specifically, the interaction between the HOMO and the electron-withdrawing group greater than the interaction between the LUMO and the electron-withdrawing group results in the orbital coefficient of the HOMO ($C_H$) of the carbon atom substituted by the electron-withdrawing group greater than the orbital coefficient of the LUMO ($C_L$). The electron-withdrawing group only needs to substitute for at least at one position, and the relationship between the total orbital coefficients ($\Sigma|C_H|$, $\Sigma|C_L|$) of the carbon bonded to the electron-withdrawing group in the mother skeleton preferably satisfies the following formula.

$$\Sigma|C_H| > \Sigma|C_L| \quad (1)$$

($C_H$: the 2PZ atomic orbital coefficient of the carbon at the substitution site in the HOMO of the mother skeleton)
($C_L$: the 2PZ atomic orbital coefficient of the carbon at the substitution site in the LUMO of the mother skeleton)

Satisfying the formula (1) means that the introduced substituent is more effective for the HOMO than the LUMO. In other words, the electron-withdrawing group introduced at a position satisfying the formula (1) can lower the HOMO more than the LUMO.

A stronger electron-withdrawing group is more desired, and more specifically the following requirement is preferably satisfied.

$$E - Eb \leq -1.51 \text{ (eV)} \quad (2)$$

In the formula (2), E denotes the LUMO level of a substituent in the mother skeleton, and Eb denotes the LUMO level of a structure in which the electron-withdrawing group is removed from the substituent. These values can be calculated using B3LYP/6-31G* and B3LYP/6-31G4, for example. The energy is expressed in eV.

(2) Shorter Wavelength due to Electron-Donating Group

Fig. 1B illustrates M(HOMO) and M(LUMO), which represent the energy of a mother skeleton alone as in Fig. 1A. On the other hand, M-D(HOMO) and M-D(LUMO) represent the energy of an organic compound with a mother skeleton into which an electron-donating group is introduced. Introduction of the electron-donating group increases the energy of HOMO and LUMO. The higher energy position is the upper side on the drawing in Fig. 1B.

In this case, a shift in LUMO greater than a shift in HOMO increases the energy difference between the HOMO and the LUMO, that is, the band gap, and shortens the emission wavelength of the organic compound.

Although satisfying the (1) has the effect of shortening the wavelength as compared with the case where no electron-withdrawing group is provided, depending on the substitution position of the electron-donating group, the wavelength may be longer than that of the mother skeleton. To make the effect of the electron-donating group contribute to a shorter wavelength, the following conditions are preferably satisfied.

The present inventors have found that the effect of an electron-donating group is indicated by an electric charge (NBO charge) determined using a natural bond orbital coefficient (NBO). The effect of an electron-donating group is more appropriately represented by the natural bond orbital coefficient than by the orbital coefficient of a molecular orbital. The requirements are as follows:

(2)-1 The electron-donating group (D) is provided on a carbon atom in a mother skeleton (M) such that a substituent can be introduced into at least one of carbon atoms on both sides of the carbon atom.
(2)-2 The electric charge value of the natural bond orbital (NBO) of the carbon atom bonded to the electron-donating group is equal to or lower than the electric charge values of the natural bond orbitals of carbon atoms on both sides of the carbon atom.
(2)-3 The electric charge value of the natural bond orbital (NBO) of the carbon atom bonded to the electron-donating group is lower by 0.02 or more than the NBO charge values of carbon atoms on both sides of the carbon atom.
(2)-1 shows a structure in which one of the adjacent carbon atoms is a carbon electron into which a substituent can be introduced. (2)-1, (2)-2, and (2)-3 mean an electron density higher than that of the adjacent carbon atoms. The following compound is an example of the mother skeleton. Table 1 shows the calculated values of the NBO charges.

[Chem. 1]

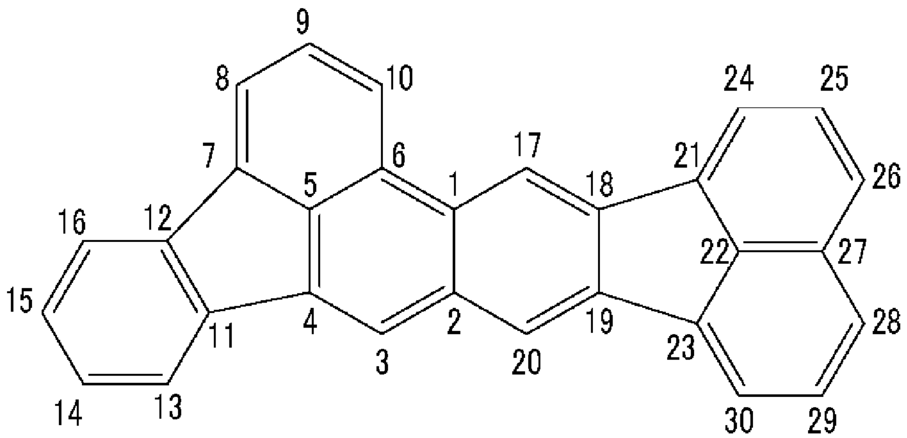

Structural Formula

[Table 1]

Table 1

| Carbon atom No. | Difference from NBO charge of adjacent carbon atom 1 | Difference from NBO charge of adjacent carbon atom 2 |
|---|---|---|
| 8 | -0.17 | 0.03 |
| 9 | -0.03 | -0.02 |
| 10 | -0.17 | 0.02 |
| 13 | -0.17 | 0.03 |
| 14 | -0.03 | 0.00 |
| 15 | 0.00 | -0.02 |
| 16 | -0.18 | 0.02 |
| 24 | -0.17 | 0.03 |
| 25 | -0.03 | -0.03 |
| 26 | 0.03 | -0.14 |
| 28 | -0.14 | 0.03 |
| 29 | -0.03 | -0.03 |
| 30 | -0.17 | 0.03 |

Carbon atoms 9, 25, and 29 satisfy the conditions (2)-1 to (2)-3. The electron-donating group substituted at these positions expands the band gap and shortens the emission wavelength. Introduction of the electron-donating group into a carbon atom with a more negative NBO charge and with a high electron density acts such that the shift in LUMO is greater than the shift in HOMO. In the present description, numerical values are rounded to two decimal places. The difference in NBO charge and the effect of shortening the wavelength have the following relationship.

[Table 2]

Table 2

| Maximum difference from NBO charge of adjacent carbon atom | Shortening of wavelength |
|---|---|
| 0.01 | Δ |
| 0.02 | ○ |
| 0.03 | ○ |

Thus, to make the emission wavelength shorter than the light emission of the mother skeleton, the electric charge value of the natural bond orbital (NBO) of the carbon atom bonded to the electron-donating group is preferably equal to or lower than the electric charge values of the natural bond orbitals of adjacent carbon atoms. Furthermore, the electric charge value of the natural bond orbital (NBO) of the carbon atom bonded to the electron-donating group is more preferably lower by 0.02 or more than one of the NBO charge values of carbon atoms on both sides of the carbon atom.

An organic compound according to an embodiment of the present invention may have an electron-withdrawing group alone, an electron-donating group alone, or both an electron-withdrawing group and an electron-donating group. To contribute to a shorter wavelength, the electron-withdrawing group preferably satisfies the (1), and the electron-donating group preferably satisfies the (2). To further shorten the emission wavelength, an electron-withdrawing group that satisfies the (1) and an electron-donating group that satisfies the (2) may be provided. This can produce an emission spectrum with a wavelength shorter than the emission wavelength of the mother skeleton itself. Furthermore, introduction of another substituent can return an emission peak with a longer wavelength to an emission peak with a shorter wavelength.

Furthermore, introduction of both an electron-withdrawing group and an electron-donating group can not only shorten the emission wavelength but also produce an organic compound with desired HOMO and LUMO by utilizing the respective effects of the electron-withdrawing group and the electron-donating group. For example, a large number of electron-withdrawing groups may be introduced to produce an organic compound with low HOMO, and conversely a large number of electron-donating groups may be introduced to produce an organic compound with high HOMO. The strength of electron withdrawal and electron donation depends on the type of substituent, and therefore the HOMO and LUMO are not influenced only by the number of substituents. As illustrated in Fig. 1D, there is also a mother skeleton without the orbital coefficient of LUMO. Fig. 1D illustrates a structure common to the exemplary compounds C34 to C38 and the LUMO orbital coefficient thereof. In this case, a shorter wavelength due to the effect of an electron-withdrawing group is observed.

An organic compound according to an embodiment of the present invention preferably further has the following structure.

(3) Containing two or more 5-membered carbon ring structures.

It is preferable to contain two or more 5-membered ring structures composed of carbon atoms. For example, it is preferable to have two or more moieties in which a fluoranthene structure can be identified as a molecular skeleton containing a 5-membered ring, as in the above structural formula.

A compound with a larger number of 5-membered carbon ring structures has higher ionization potential. An organic compound with high ionization potential is resistant to oxidation and is highly stable against oxidation. Thus, it is preferable to have two or more 5-membered carbon ring structures to improve the stability of the organic compound.

(4) A bond between a mother skeleton and a substituent is a carbon-carbon bond.

When an electron-withdrawing group or an electron-donating group is bonded to a mother skeleton, the bond is preferably a carbon-carbon bond. This is because the carbon-carbon bond is a stronger bond than other bonds. Thus, a bond between a mother skeleton and a substituent is preferably a carbon-carbon bond to improve the stability of the organic compound. Fig. 1E illustrates the energy of each bond.

Fig. 1E is a diagram of chemical bonds and their binding energies. A carbon-carbon bond has higher binding energy than heteroatom-carbon bonds, such as a nitrogen-carbon bond. In a light-emitting layer of an organic light-emitting element, excitons are continuously generated at high density and emit light. An organic compound that can withstand cycles of excitation and light emission is therefore required. A carbon atom-carbon atom bond is more resistant to cleavage than heteroatom-carbon atom bonds due to its higher binding energy and is more resistant to excited state degradation during repeated cycles of photoexcitation and light emission of an organic compound. The excited state degradation refers to the decomposition or modification of an organic compound due to the dissociation of a bond in the organic compound.

An example of the organic compound according to the present embodiment is described below. However, the present invention is not limited to the example. Substituents on a mother skeleton are an electron-withdrawing group (A), an electron-donating group (D), and a substituent (X). The substituent (X) refers to a substituent selected from electron-withdrawing groups and electron-donating groups.

[Chem. 2]

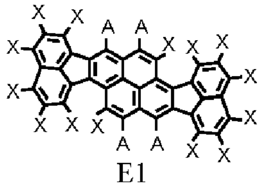
E1
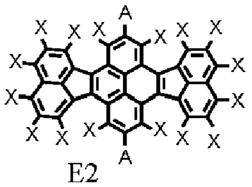
E2
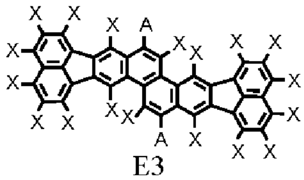
E3
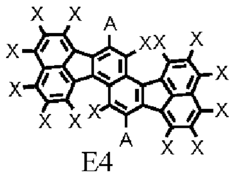
E4
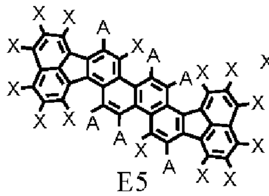
E5
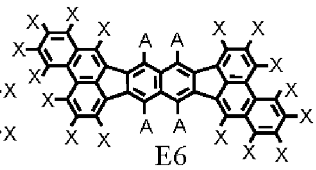
E6
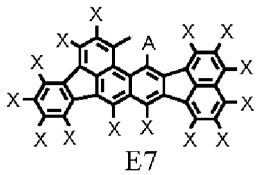
E7
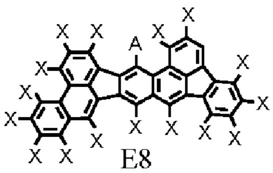
E8
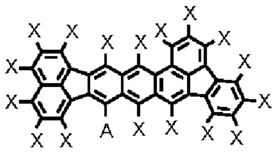
E9
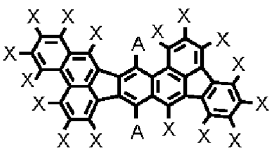
E10
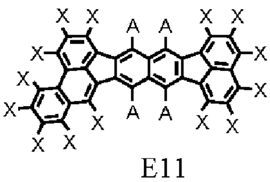
E11
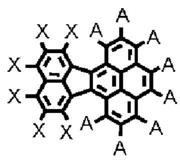
E12

A in E1 to E12 represents a substitution position of an electron-withdrawing group to shorten the wavelength. The substitution site of A is a site that satisfies the relationship of the formula (1) in molecular orbital calculation. At this time, at least one of the substituents X in E1 to E12 may be substituted with an electron-donating group.

[Chem. 3]

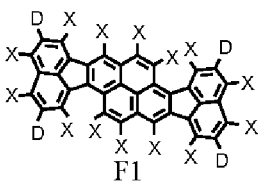
F1
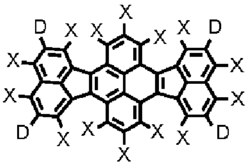
F2
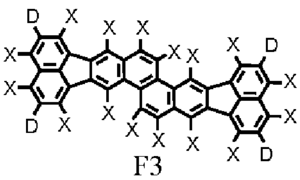
F3
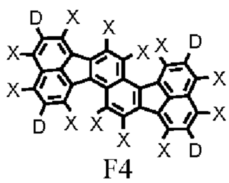
F4
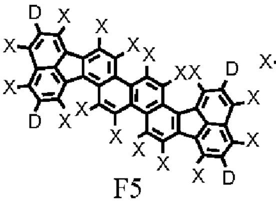
F5
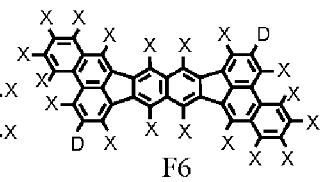
F6
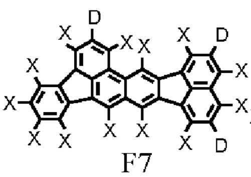
F7
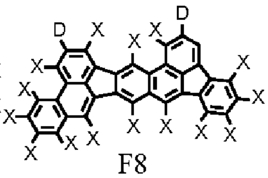
F8
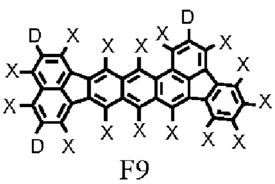
F9
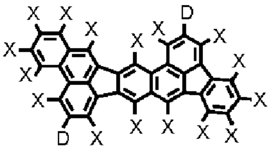
F10
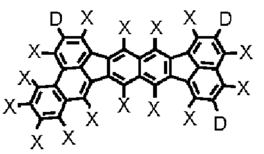
F11
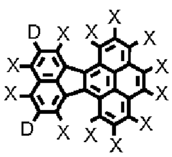
F12

D in F1 to F12 represents a substitution position of an electron-donating group to shorten the wavelength. The

substitution site of D refers to a substitution position satisfying the requirements of (2)-1 and (2)-2, as in the positions of carbon atom Nos. 9, 25, and 29 in Table 1. At this time, at least one of the substituents X in F1 to F12 may be an electron-withdrawing group.

[Chem. 4]

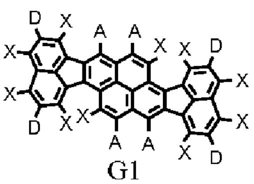
G1
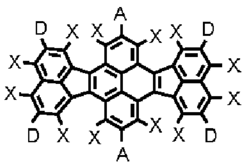
G2
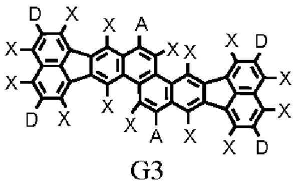
G3
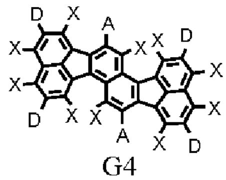
G4

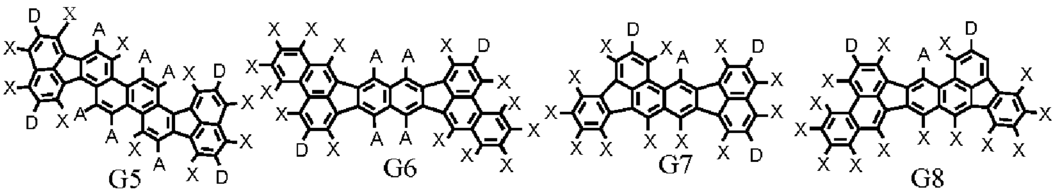
G5 G6 G7 G8

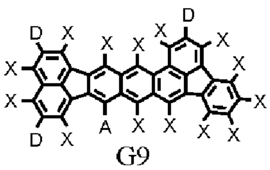
G9
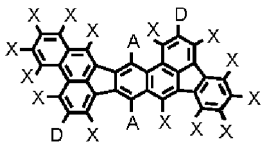
G10
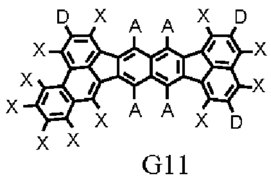
G11
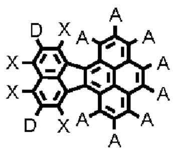
G12

In G1 to G12, A represents a substitution position of an electron-withdrawing group to shorten the wavelength, and D represents a substitution position of an electron-donating group to shorten the wavelength. The substitution site of A is a site that satisfies the relationship of the formula (1) in molecular orbital calculation. The substitution site of D refers to a substitution position satisfying the requirements of (2)-1 and (2)-2, as in the positions of carbon atom Nos. 9, 25, and 29 in Table 1.

The substituents in E1 to E12, F1 to F12, and G1 to G12 are described below.

The electron-withdrawing group A is preferably one of a cyano group, a halogen atom, such as fluorine, chlorine, or bromine, an alkyl group having a halogen atom as a substituent, such as a trifluoromethyl group, and an aryl group or a heterocyclic group having these as a substituent.

An aryl group of the electron-withdrawing group A may be an aryl group having 6 to 18 carbon atoms. Specific examples include, but are not limited to, a phenyl group, a naphthyl group, an indenyl group, a biphenyl group, a terphenyl group, and a fluorenyl group.

A heterocyclic group of the electron-withdrawing group A may be a heterocyclic group having at least one of nitrogen, oxygen, and sulfur as a heteroatom and having 3 to 15 carbon atoms. Specific examples include, but are not limited to, a pyridyl group, an oxazolyl group, an oxadiazolyl group, a thiazolyl group, a thiadiazolyl group, a carbazolyl group, an acridinyl group, and a phenanthrolyl group.

An electron-donating group D may be a hydrogen atom, an alkyl group, an alkoxy group, an amino group, or an aryl or heterocyclic group substituted therewith.

An alkyl group of the electron-donating group D may be an alkyl group having 1 to 10 carbon atoms. Specific examples include, but are not limited to, a methyl group, an ethyl group, a n-propyl group, an isopropyl group, a n-butyl group, a t-butyl group, a sec-butyl group, an octyl group, a cyclohexyl group, a 1-adamantyl group, and a 2-adamantyl group.

An alkoxy group of the electron-donating group D may be an alkoxy group having 1 to 10 carbon atoms. Specific examples include, but are not limited to, a methoxy group, an ethoxy group, a propoxy group, a 2-ethyl-sixyloxy group, and a benzyloxy group.

An aryl group and a heterocyclic group of the electron-donating group D are the same as the examples of the electron-withdrawing group. Because the aryl group or the heterocyclic group has a different substituent, the electron-withdrawing and electron-donating properties are different.

An amino group of the electron-donating group D may be an amino group having an alkyl group or an aryl group as a substituent. The alkyl group and the aryl group may be the same as the substituents exemplified as the

electron-donating group D. Specific examples include, but are not limited to, an N-methylamino group, an N-ethylamino group, an N,N-dimethylamino group, an N,N-diethylamino group, an N-methyl-N-ethylamino group, an N-benzylamino group, an N-methyl-N-benzylamino group, an N,N-dibenzylamino group, an anilino group, an N,N-diphenylamino group, an N,N-dinaphthylamino group, an N,N-difluorenylamino group, an N-phenyl-N-tolylamino group, an N,N-ditolylamino group, an N-methyl-N-phenylamino group, an N,N-dianisolylamino group, an N-mesityl-N-phenylamino group, an N,N-dimesitylamino group, an N-phenyl-N-(4-t-butylphenyl)amino group, an N-phenyl-N-(4-trifluoromethylphenyl)amino group, and an N-piperidyl group.

Examples of the substituent that the alkyl group, the alkoxy group, the aryl group, the heterocyclic group, and the amino group may further have include, but are not limited to, alkyl groups having 1 to 10 carbon atoms, such as a methyl group, an ethyl group, a n-propyl group, an isopropyl group, a n-butyl group, and a tert-butyl group, aralkyl groups, such as a benzyl group, aryl groups having 6 to 18 carbon atoms, such as a phenyl group and a biphenyl group, heterocyclic groups having at least one of nitrogen, oxygen, and sulfur as a heteroatom and having 3 to 15 carbon atoms, such as a pyridyl group and a pyrrolyl group, amino groups having an alkyl group or an aryl group as a substituent, such as a dimethylamino group, a diethylamino group, a dibenzylamino group, a diphenylamino group, and a ditolylamino group, alkoxy groups having 1 to 10 carbon atoms, such as a methoxy group, an ethoxy group, and a propoxy group, and aryloxy groups, such as a phenoxy group. Among these, alkyl groups, aralkyl groups, and aryl groups are preferred.

The substituents X are independently selected from a hydrogen atom, an alkyl group, an alkoxy group, an aryl group, a heterocyclic group, an aryloxy group, and a cyano group. The alkyl group may be an alkyl group having 1 to 10 carbon atoms. Specific examples include a methyl group, an ethyl group, a n-propyl group, an isopropyl group, a n-butyl group, and a t-butyl group. The aryl group may be an aryl group having 6 to 18 carbon atoms. Specific examples include a phenyl group, a naphthyl group, an indenyl group, a biphenyl group, a terphenyl group, and a fluorenyl group. A heterocyclic group having at least one of nitrogen, oxygen, and sulfur as a heteroatom and having 3 to 15 carbon atoms may also be used. Specific examples include a pyridyl group, an oxazolyl group, an oxadiazolyl group, a thiazolyl group, a thiadiazolyl group, a carbazolyl group, an acridinyl group, and a phenanthrolyl group.

In E1 to E12, F1 to F12, and G1 to G12, an electron-withdrawing group is preferably introduced only at the position A. In E1 to E12, F1 to F12, and G1 to G12, an electron-donating group is preferably introduced only at the position D.

An electron-withdrawing group and an electron-donating group introduced at a position other than these positions may have a small effect of shortening the emission wavelength of the organic compound.

Specific examples of the organic compound according to the present embodiment are described below. The following examples are specific examples of G1 to G12. An organic compound according to an embodiment of the present invention can also be produced by introducing a substituent into the groups E and F as in the following exemplary compounds. However, the present invention is not limited to these examples.

[Chem. 5]

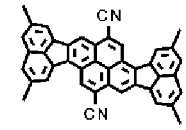
A1

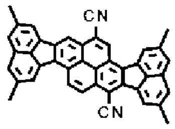
A2

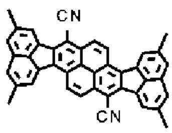
A3

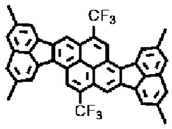
A4

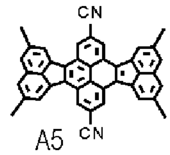
A5

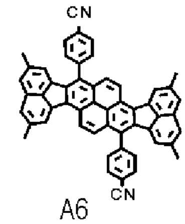
A6

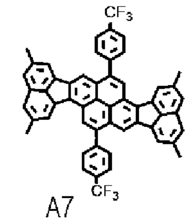
A7

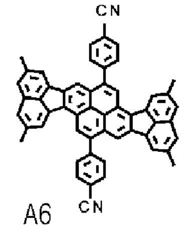
A6

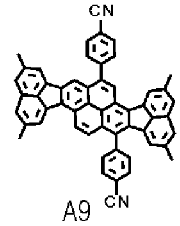
A9

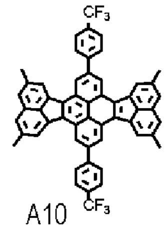
A10

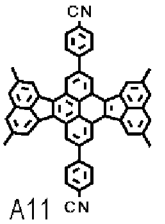
A11

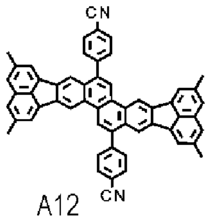
A12

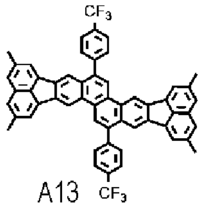
A13

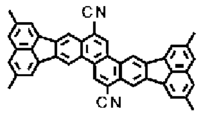
A14

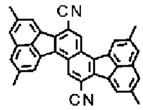
A15

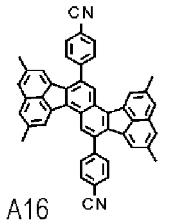
A16

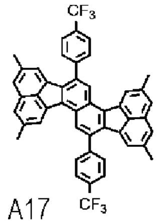
A17

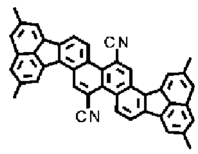
A18

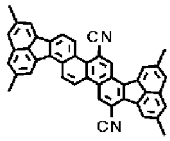
A19

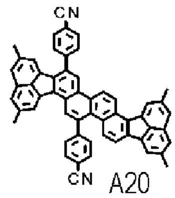
A20

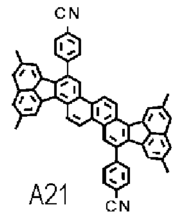
A21

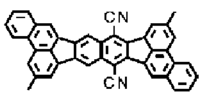
A22

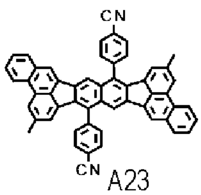
A23

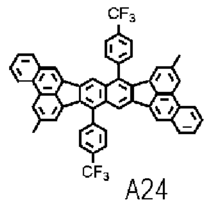
A24

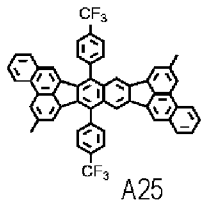
A25

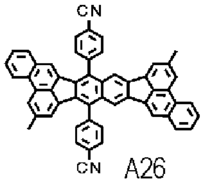
A26

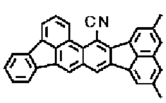
A27

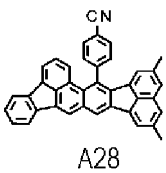
A28

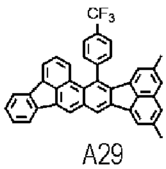
A29

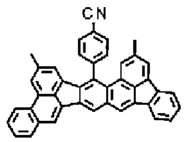
A30

[Chem. 6]

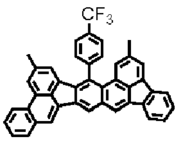
A31

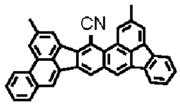
A32

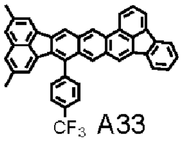
A33

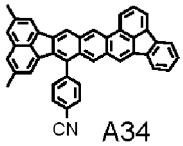
A34

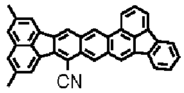
A35

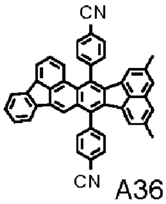
A36

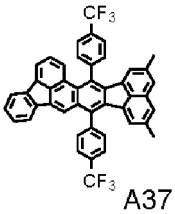
A37

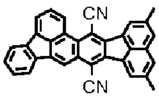
A38

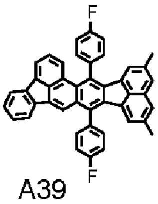
A39

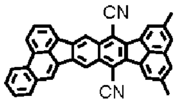
A40

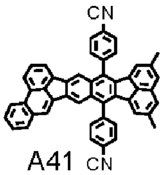
A41

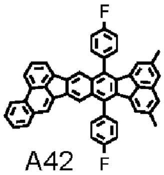
A42

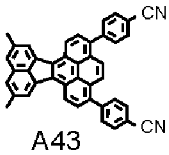
A43

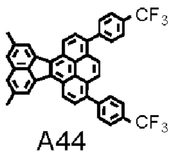
A44

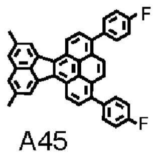
A45

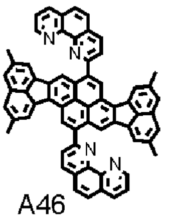
A46

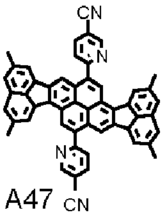
A47

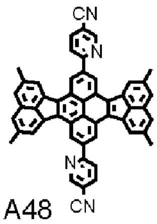
A48

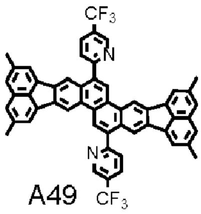
A49

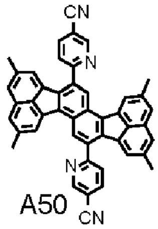
A50

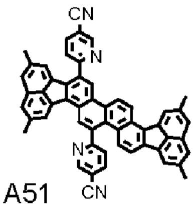
A51

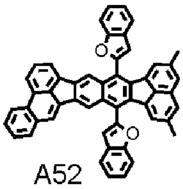
A52

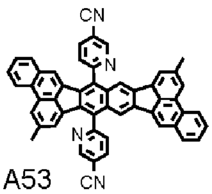
A53

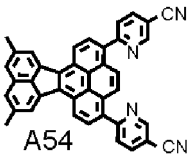
A54

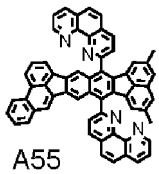
A55

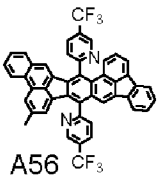
A56

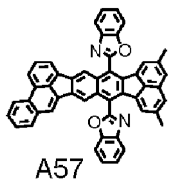
A57

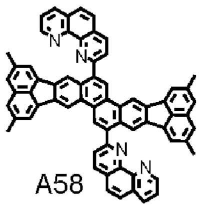
A58

[Chem. 7]

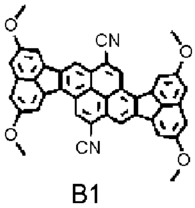
B1

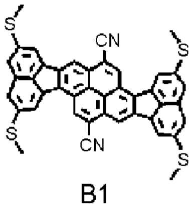
B1

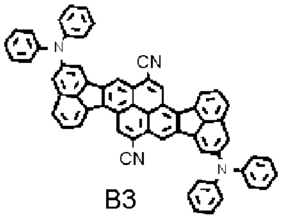
B3

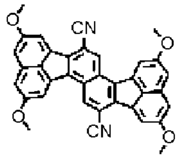
B4

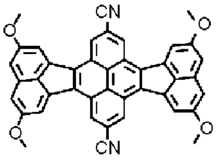
B5

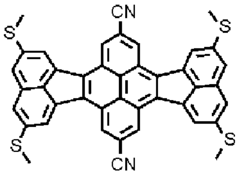
B6

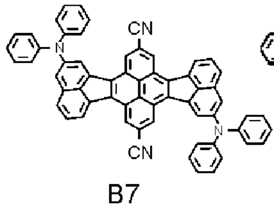
B7

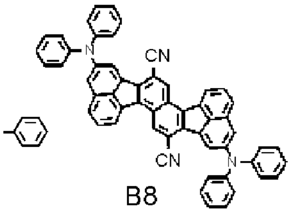
B8

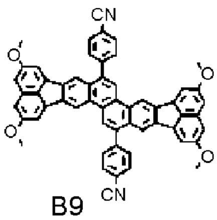
B9

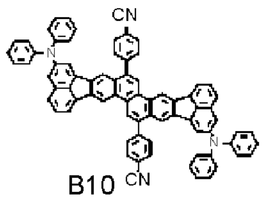
B10

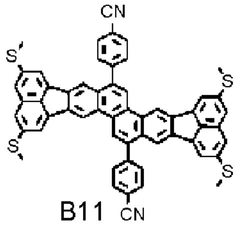
B11

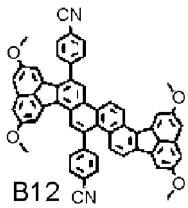
B12

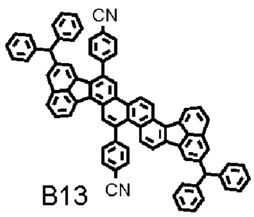
B13

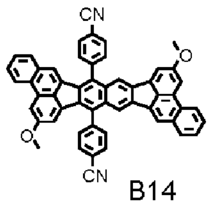
B14

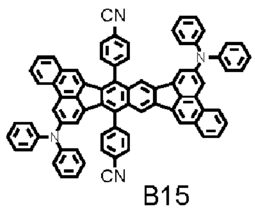
B15

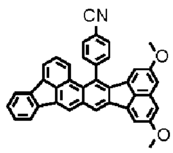
B16

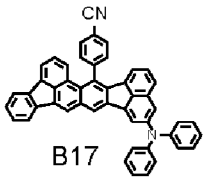
B17

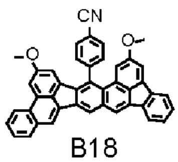
B18

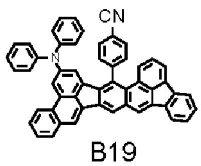
B19

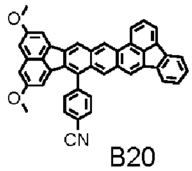
B20

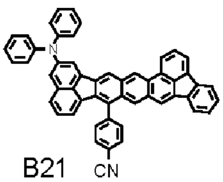
B21

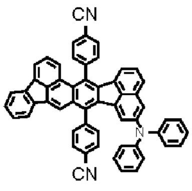
B22

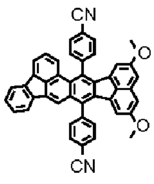
B23

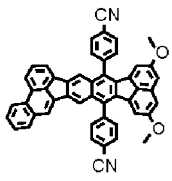
B24

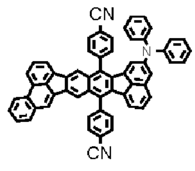
B25

Organic compounds in the group A have at least one 5-membered ring and are stable against oxidation. A mother skeleton and a substituent are via a carbon-carbon bond. Thus, among the compounds according to the present embodiment, the exemplary compounds belonging to the group A are preferred in terms of high molecular stability. The organic compounds of the group A can be used for a light-emitting layer host material, a transport layer, and an injection layer, as well as a light-emitting material.

Organic compounds of the group B are organic compounds that have a heteroatom-carbon bond as a bond between a basic skeleton and a substituent. These exemplary compounds are less stable than the organic compounds

of the group A but have high electron-donating and electron-withdrawing effects.

[Organic Light-Emitting Element]

An organic light-emitting element according to an embodiment of the present invention is described below.

An organic light-emitting element according to the present embodiment includes at least a pair of electrodes, a positive electrode and a negative electrode, and an organic compound layer between the electrodes. In the organic light-emitting element according to the present embodiment, the organic compound layer may be a single layer or a laminate of a plurality of layers, provided that the organic compound layer has a light-emitting layer.

When the organic compound layer is a laminate of a plurality of layers, the organic compound layer may have a hole-injection layer, a hole-transport layer, an electron-blocking layer, a hole/exciton-blocking layer, an electron-transport layer, and/or an electron-injection layer, in addition to the light-emitting layer. The light-emitting layer may be a single layer or a laminate of a plurality of layers.

Specific structures of an organic light-emitting element may be the following (1) to (6).

(1) (Substrate/)positive electrode/light-emitting layer/electron-injection layer/negative electrode
(2) (Substrate/)positive electrode/hole-transport layer/electron-transport layer/electron-injection layer/negative electrode
(3) (Substrate/)positive electrode/hole-transport layer/light-emitting layer/electron-transport layer/electron-injection layer/negative electrode
(4) (Substrate/)positive electrode/hole-injection layer/hole-transport layer/light-emitting layer/electron-transport layer/electron-injection layer/negative electrode
(5) (Substrate/)positive electrode/hole-transport layer/light-emitting layer/blocking layer/electron-transport layer/electron-injection layer/negative electrode
(6) (Substrate/)positive electrode/hole-injection layer/hole-transport layer/light-emitting layer/blocking layer/electron-transport layer/electron-injection layer/negative electrode

In the organic light-emitting element according to the present embodiment, at least one layer of the organic compound layers contains the organic compound according to the present embodiment. More specifically, the organic compound according to the present embodiment is contained in one of the hole-injection layer, the hole-transport layer, the electron-blocking layer, the light-emitting layer, the hole/exciton-blocking layer, the electron-transport layer, the electron-injection layer, and the like. The organic compound according to the present embodiment is preferably contained in the light-emitting layer.

In the organic light-emitting element according to the present embodiment, when the organic compound according to the present embodiment is contained in the light-emitting layer, the light-emitting layer may contain a first compound and a second compound. The first compound may be the organic compound according to the present embodiment, and the light-emitting layer may be a layer composed only of the first compound or a mixture layer containing the first compound and a second compound different from the first compound. When the light-emitting layer is a mixture layer of the first compound and the second compound, the weight ratio of the first compound may be smaller than the weight ratio of the second compound. The denominator of the weight ratio may be the whole compounds constituting the light-emitting layer.

Thus, the organic compound according to the present embodiment may be used as a host or guest in the light-emitting layer. In particular, the organic compound according to the present embodiment is preferably used as a guest. The organic compound may also be used as an assist material that may be contained in the light-emitting layer. The host is the compound with the highest mass ratio among the compounds constituting the light-emitting layer. The guest is a compound that has a lower mass ratio than the host among the compounds constituting the light-emitting layer and that is a principal light-emitting compound. The assist material is a compound that has a lower mass ratio than the host among the compounds constituting the light-emitting layer and that assists the guest in emitting light. The assist material is also referred to as a second host.

When the organic compound according to the present embodiment is used as a guest in the light-emitting layer, the concentration of the guest preferably ranges from 0.01% to 20% by mass, more preferably 0.1% to 5% by mass, of the entire light-emitting layer.

When the organic compound according to the present embodiment is used as a guest in the light-emitting layer, a material with higher LUMO than the organic compound according to the present embodiment (a material with LUMO closer to the vacuum level) is preferably used as a host. This is because the use of the material with higher LUMO than the organic compound according to the present embodiment as a host enables the organic compound according to the present embodiment to more effectively receive electrons supplied to the host in the light-emitting layer. The use of the organic compound according to the present embodiment as a guest material can further improve chromaticity during

light emission. For example, shortening a wavelength can bring an emission spectrum of a basic skeleton closer to the blueness of sRGB of blue light emission and expand the color reproduction range.

The organic compound according to the present embodiment is used as a host or guest in the light-emitting layer, particularly as a guest in the light-emitting layer. The light-emitting layer may be composed of a single layer or multiple layers or may contain a light-emitting material of another emission color. The term "multiple layers", as used herein, refers to a laminate of a light-emitting layer and another light-emitting layer. In such a case, the organic light-emitting element may have any emission color. More specifically, the emission color of the organic light-emitting element is not limited to blue and may be white or neutral color. For white color emission, another light-emitting layer emits light of a color other than blue, such as red or green. Furthermore, each light-emitting layer may emit light of blue, green, or red. With respect to a film-forming method, at least a light-emitting layer is preferably formed by a vacuum evaporation method.

The organic compound according to the present embodiment can be used as a constituent material of an organic compound layer other than the light-emitting layer constituting the organic light-emitting element according to the present embodiment. More specifically, the organic compound according to the present embodiment may be used as a constituent material of an electron-transport layer, an electron-injection layer, a hole-transport layer, a hole-injection layer, and/or a hole-blocking layer.

If necessary, the organic compound according to the present embodiment may be used in combination with a known low-molecular-weight or high-molecular-weight hole-injection compound or hole-transport compound, host compound, light-emitting compound, electron-injection compound, or electron-transport compound. Examples of these compounds are described below.

The hole-injection or hole-transport material is preferably a material that can facilitate the injection of holes from a positive electrode and that has high hole mobility to transport the injected holes to a light-emitting layer. To reduce degradation of film quality, such as crystallization, in the organic light-emitting element, a material with a high glass transition temperature is preferred. Examples of the low-molecular-weight or high-molecular-weight material with hole injection or transport ability include, but are not limited to, triarylamine derivatives, aryl carbazole derivatives, phenylenediamine derivatives, stilbene derivatives, phthalocyanine derivatives, porphyrin derivatives, polyvinylcarbazole, polythiophene, and other electrically conductive polymers. The hole-injection or hole-transport material is also suitable for an electron-blocking layer. Specific examples of compounds that can be used as the hole-injection or hole-transport material are described below. As a matter of course, the present invention is not limited to these examples.

[Chem. 8]

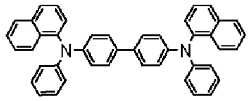

HT1

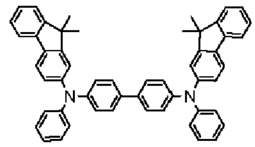

HT2

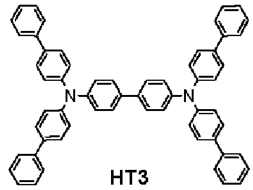

HT3

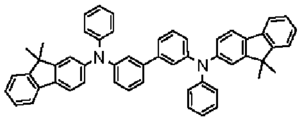

HT4

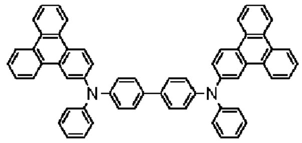

HT5

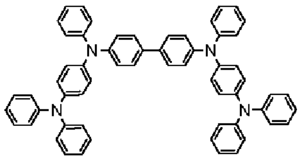

HT6

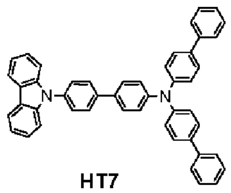

HT7

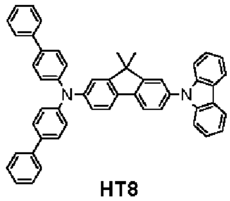

HT8

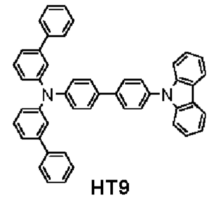

HT9

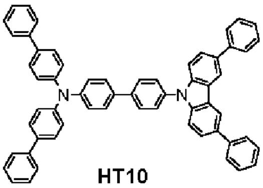

HT10

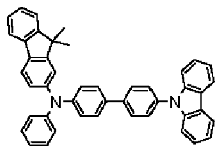

HT11

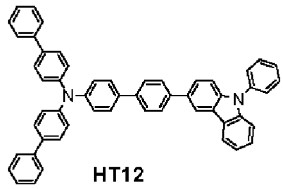

HT12

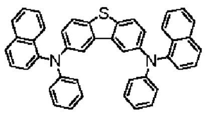

HT13

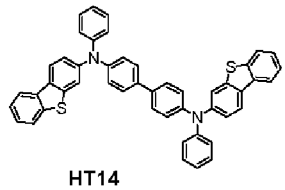

HT14

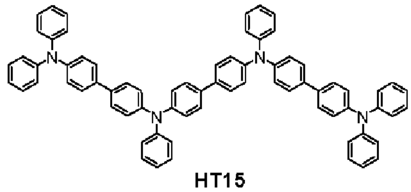

HT15

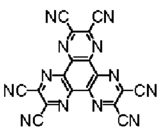

HT16

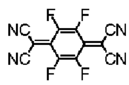

HT17

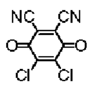

HT18

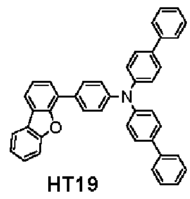

HT19

Examples of a light-emitting material mainly related to the light-emitting function include, in addition to the organic compounds represented by the general formula [1], fused-ring compounds (for example, fluorene derivatives, naphthalene derivatives, pyrene derivatives, perylene derivatives, tetracene derivatives, anthracene derivatives, rubrene, etc.), quinacridone derivatives, coumarin derivatives, stilbene derivatives, organoaluminum complexes, such as tris(8-quinolinolato)aluminum, iridium complexes, platinum complexes, rhenium complexes, copper complexes, europium complexes, ruthenium complexes, and polymer derivatives, such as poly(phenylene vinylene) derivatives, polyfluorene derivatives, and polyphenylene derivatives.

When a mixture layer of the organic compound according to the present embodiment and another light-emitting material is formed, or when light-emitting layers are laminated, the other light-emitting material preferably has low HOMO/LUMO energy. This is because high HOMO/LUMO energy may result in the formation of a quenching component or a trap level, such as the formation of an exciplex with the organic compound according to the present embodiment.

Specific examples of compounds that can be used as light-emitting materials are described below. However, the present invention is not limited to these examples.

[Chem. 9]

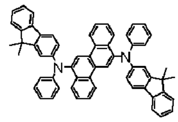
BD1
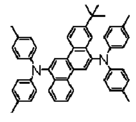
BD2
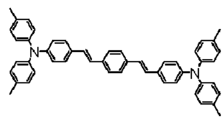
BD3
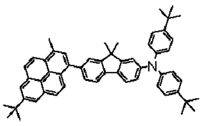
BD4
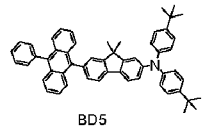
BD5
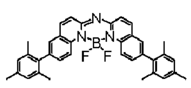
BD6
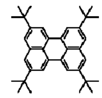
BD7
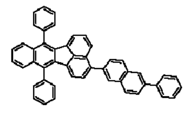
BD8
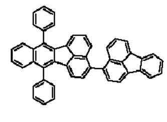
BD9
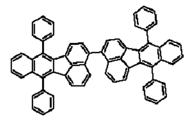
BD10
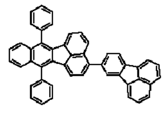
BD11
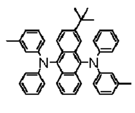
GD1
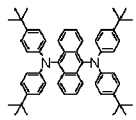
GD2
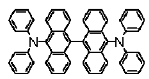
GD3
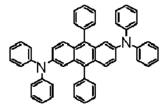
GD4
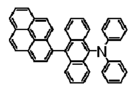
GD5
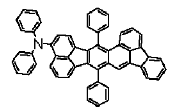
GD6
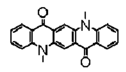
GD7
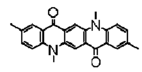
GD8
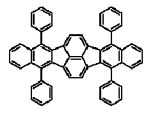
GD9
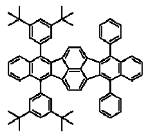
GD10
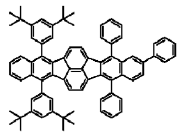
GD11
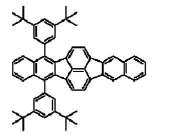
GD12
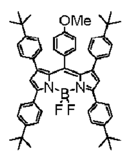
RD1
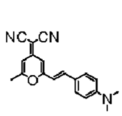
RD2
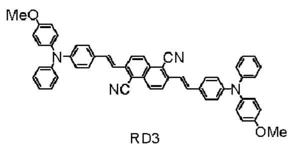
RD3
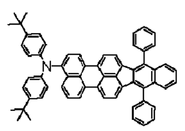
RD4
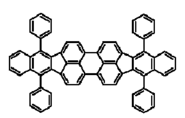
RD5
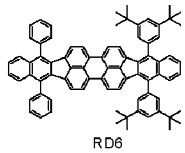
RD6
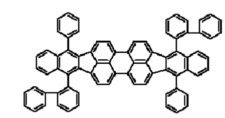
RD7
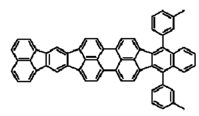
RD8

Examples of a light-emitting layer host or a light-emitting assist material in a light-emitting layer include aromatic hydrocarbon compounds and derivatives thereof, carbazole derivatives, dibenzofuran derivatives, dibenzothiophene derivatives, organoaluminum complexes, such as tris(8-quinolinolato)aluminum, and organic beryllium complexes. The host material particularly preferably has an anthracene, tetracene, perylene, or pyrene skeleton in the molecular skeleton. This is because such a host material is composed of carbon and hydrogen as described above and has S1 energy required for sufficient energy transfer to the organic compound according to the present embodiment. Specific examples of a compound used as a light-emitting layer host or a light-emitting assist material in a light-emitting layer are described below. However, the present invention is not limited to these examples.

[Chem. 10]

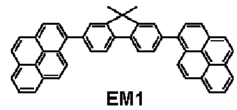
EM1
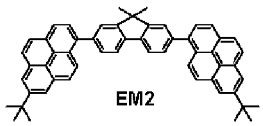
EM2
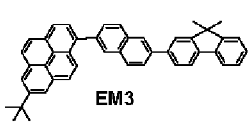
EM3
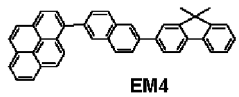
EM4
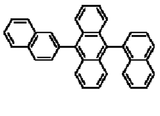
EM5
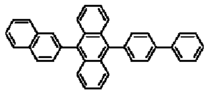
EM6
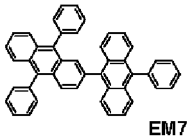
EM7
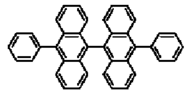
EM8
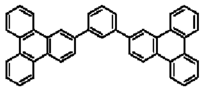
EM9
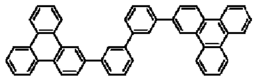
EM10
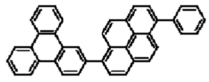
EM11
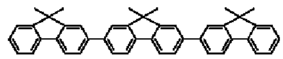
EM12
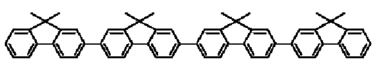
EM13
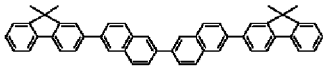
EM14
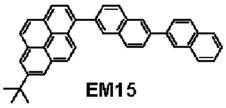
EM15
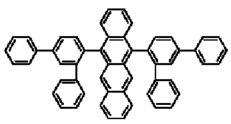
EM16
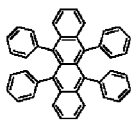
EM17
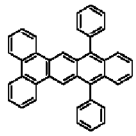
EM18
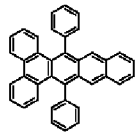
EM19
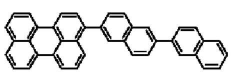
EM20
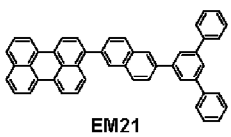
EM21
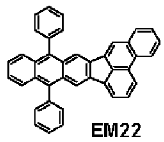
EM22
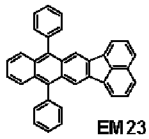
EM23
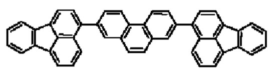
EM24
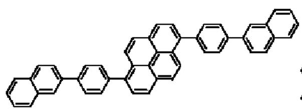
EM25
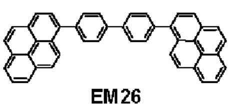
EM26
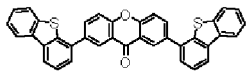
EM27
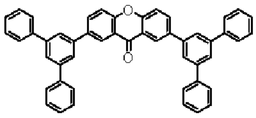
EM28
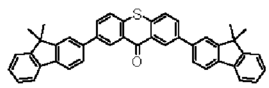
EM29
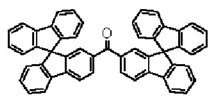
EM30
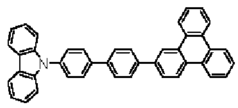
EM31
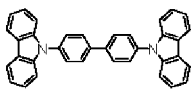
EM32
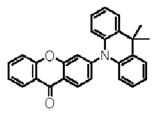
EM33
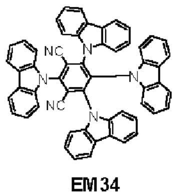
EM34
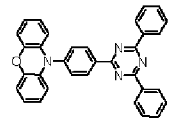
EM35

An electron-transport material can be selected from materials that can transport electrons injected from a negative electrode to a light-emitting layer and is selected in consideration of the balance with the hole mobility of a hole-transport material and the like. Examples of materials with electron-transport ability include, but are not limited to, oxadiazole derivatives, oxazole derivatives, pyrazine derivatives, triazole derivatives, triazine derivatives, quinoline derivatives, quinoxaline derivatives, phenanthroline derivatives, organoaluminum complexes, and fused-ring compounds (for example, fluorene derivatives, naphthalene derivatives, chrysene derivatives, and anthracene derivatives). Furthermore, the electron-transport material is also suitable for a hole-blocking layer. Specific examples of compounds that can be used as electron-transport materials are described below. However, the present invention is not limited to these

examples.

[Chem. 11]

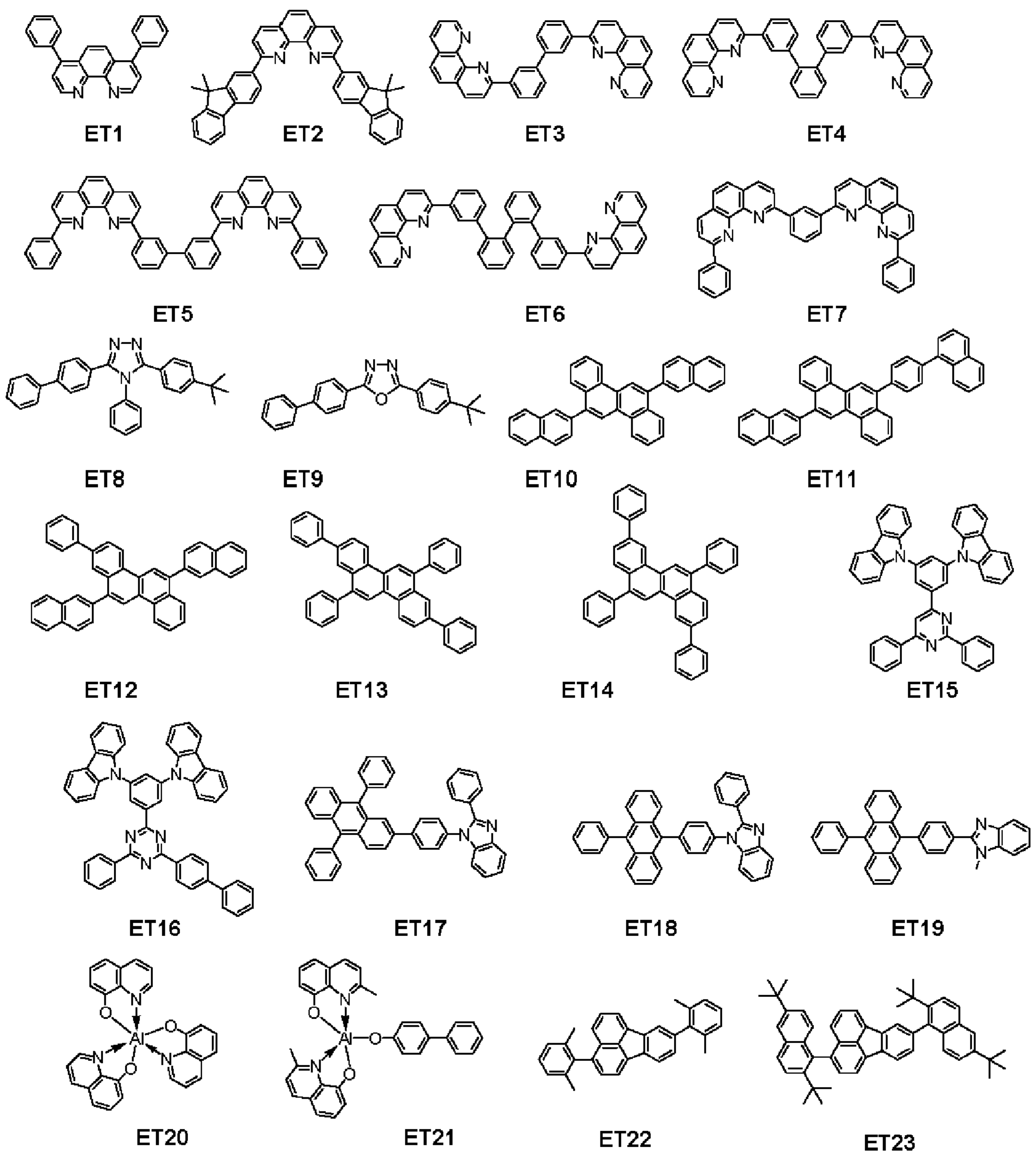

ET1 ET2 ET3 ET4

ET5 ET6 ET7

ET8 ET9 ET10 ET11

ET12 ET13 ET14 ET15

ET16 ET17 ET18 ET19

ET20 ET21 ET22 ET23

<Structure of Organic Light-Emitting Element>

An organic light-emitting element includes a positive electrode, an organic compound layer, and a negative electrode on a substrate. A protective layer, a color filter, or the like may be provided on the negative electrode. When a color filter is provided, a planarization layer may be provided between the color filter and a protective layer. The planarization layer may be composed of an acrylic resin or the like.

[Substrate]

The substrate may be formed of quartz, glass, silicon wafer, resin, metal, or the like. The substrate may have a switching element, such as a transistor, and a wire, on which an insulating layer may be provided. The insulating layer may be composed of any material, provided that the insulating layer can have a contact hole to ensure electrical connection between the positive electrode and the wire and can be insulated from unconnected wires. For example, the insulating

layer may be formed of a resin, such as polyimide, silicon oxide, or silicon nitride.

[Electrode]

A pair of electrodes can be used as electrodes. The pair of electrodes may be a positive electrode and a negative electrode. When an electric field is applied in a direction in which the organic light-emitting element emits light, an electrode with a high electric potential is a positive electrode, and the other electrode is a negative electrode. In other words, the electrode that supplies holes to the light-emitting layer is a positive electrode, and the electrode that supplies electrons is a negative electrode.

A constituent material of the positive electrode preferably has as large a work function as possible. Examples of the constituent material include metal elements, such as gold, platinum, silver, copper, nickel, palladium, cobalt, selenium, vanadium, and tungsten, mixtures thereof, alloys thereof, and metal oxides, such as tin oxide, zinc oxide, indium oxide, indium tin oxide (ITO), and indium zinc oxide. Electrically conductive polymers, such as polyaniline, polypyrrole, and polythiophene, may also be used.

These electrode materials may be used alone or in combination. The positive electrode may be composed of a single layer or a plurality of layers.

When used as a reflective electrode, for example, chromium, aluminum, silver, titanium, tungsten, molybdenum, an alloy thereof, or a laminate thereof can be used. When used as a transparent electrode, an oxide transparent conductive layer, such as indium tin oxide (ITO) or indium zinc oxide, can be used. However, the present invention is not limited thereto. The electrodes may be formed by photolithography.

A constituent material of the negative electrode is preferably a material with a small work function. For example, an alkali metal, such as lithium, an alkaline-earth metal, such as calcium, a metal element, such as aluminum, titanium, manganese, silver, lead, or chromium, or a mixture thereof may be used. An alloy of these metal elements may also be used. For example, magnesium-silver, aluminum-lithium, aluminum-magnesium, silver-copper, or zinc-silver may be used. A metal oxide, such as indium tin oxide (ITO), may also be used. These electrode materials may be used alone or in combination. The negative electrode may be composed of a single layer or a plurality of layers. Among them, silver is preferably used, and a silver alloy is more preferably used to reduce the aggregation of silver. As long as the aggregation of silver can be reduced, the alloy may have any ratio. For example, it may be 1:1.

The negative electrode may be an oxide conductive layer, such as ITO, for a top emission element or may be a reflective electrode, such as aluminum (Al), for a bottom emission element and is not particularly limited. The negative electrode may be formed by any method. A direct-current or alternating-current sputtering method is preferably used to achieve good film coverage and easily decrease resistance.

[Protective Layer]

A protective layer may be provided on the negative electrode. For example, a glass sheet with a moisture absorbent may be attached to the negative electrode to decrease the amount of water or the like entering the organic compound layer and reduce the occurrence of display defects. In another embodiment, a passivation film, such as silicon nitride, may be provided on the negative electrode to decrease the amount of water or the like entering the organic compound layer. For example, after the negative electrode is formed, the negative electrode is transferred to another chamber without breaking the vacuum, and a silicon nitride film with a thickness of 2 $\mu$m may be formed as a protective layer by a CVD method. The protective layer may be formed by the CVD method followed by an atomic layer deposition (ALD) method.

[Color Filter]

A color filter may be provided on the protective layer. For example, a color filter that matches the size of the organic light-emitting element may be provided on another substrate and may be bonded to the substrate on which the organic light-emitting element is provided, or a color filter may be patterned on the protective layer by photolithography. The color filter may be composed of a polymer.

[Planarization Layer]

A planarization layer may be provided between the color filter and the protective layer. The planarization layer may be composed of an organic compound and is preferably composed of a high-molecular-weight compound, though it may be composed of a low-molecular-weight compound.

The planarization layer may be provided above and below the color filter, and the constituent materials thereof may be the same or different. Specific examples include polyvinylcarbazole resins, polycarbonate resins, polyester

resins, ABS resins, acrylic resins, polyimide resins, phenolic resins, epoxy resins, silicon resins, and urea resins.

[Opposite Substrate]

An opposite substrate may be provided on the planarization layer. The opposite substrate is so called because it is provided at a position corresponding to the substrate. The opposite substrate may be composed of the same material as the substrate.

[Organic Layer]

An organic compound layer (a hole-injection layer, a hole-transport layer, an electron-blocking layer, a light-emitting layer, a hole-blocking layer, an electron-transport layer, an electron-injection layer, etc.) constituting an organic light-emitting element according to an embodiment of the present invention is formed by the following method.

An organic compound layer constituting an organic light-emitting element according to an embodiment of the present invention can be formed by a dry process, such as a vacuum evaporation method, an ionized deposition method, sputtering, or plasma. Instead of the dry process, a wet process may also be employed in which a layer is formed by a known coating method (for example, spin coating, dipping, a casting method, an LB method, an ink jet method, etc.) using an appropriate solvent.

A layer formed by a vacuum evaporation method, a solution coating method, or the like undergoes little crystallization or the like and has high temporal stability. When a film is formed by a coating method, the film may also be formed in combination with an appropriate binder resin.

Examples of the binder resin include, but are not limited to, polyvinylcarbazole resins, polycarbonate resins, polyester resins, ABS resins, acrylic resins, polyimide resins, phenolic resins, epoxy resins, silicon resins, and urea resins.

These binder resins may be used alone as a homopolymer or a copolymer or may be used in combination. If necessary, an additive agent, such as a known plasticizer, oxidation inhibitor, and/or ultraviolet absorbent, may also be used.

<Applications of Organic Light-Emitting Element according to Embodiment of Present Invention>

An organic light-emitting element according to an embodiment of the present invention can be used as a constituent of a display apparatus or a lighting apparatus. Other applications include an exposure light source of an electrophotographic image-forming apparatus, a backlight of a liquid crystal display, and a light-emitting apparatus with a color filter in a white light source.

The display apparatus may include an image input unit for inputting image information from an area CCD, a linear CCD, a memory card, or the like, may include an information processing unit for processing the input information, and may be an image-information-processing apparatus for displaying an input image on a display unit.

A display unit of an imaging apparatus or an ink jet printer may have a touch panel function. A driving system of the touch panel function may be, but is not limited to, an infrared radiation system, an electrostatic capacitance system, a resistive film system, or an electromagnetic induction system. The display apparatus may be used for a display unit of a multifunction printer.

Next, the display apparatus according to the present embodiment is described with reference to the accompanying drawings.

Figs. 2A and 2B are schematic cross-sectional views of a display apparatus that includes an organic light-emitting element and a transistor coupled to the organic light-emitting element. The transistor is an example of an active element. The transistor may be a thin-film transistor (TFT).

Fig. 2A illustrates a pixel serving as a constituent of the display apparatus according to the present embodiment. The pixel has subpixels 10. The subpixels are divided into 10R, 10G, and 10B according to light emission. The emission colors may be distinguished by the wavelength emitted from the light-emitting layer, or light emitted from each subpixel may be selectively transmitted or color-converted with a color filter or the like. Each subpixel has, on an interlayer insulating layer 1, a reflective electrode 2 as a first electrode, an insulating layer 3 covering the ends of the reflective electrode 2, organic compound layers 4 covering the first electrode and the insulating layer, a transparent electrode 5, a protective layer 6, and a color filter 7.

A transistor and/or a capacitor element may be provided under or inside the interlayer insulating layer 1. The transistor may be electrically connected to the first electrode via a contact hole (not shown) or the like.

The insulating layer 3 is also referred to as a bank or a pixel separation film. The insulating layer 3 covers the ends of the first electrode and surrounds the first electrode. A portion of the first electrode not covered with the insulating layer is in contact with the organic compound layers 4 and serves as a light-emitting region.

The organic compound layers 4 include a hole-injection layer 41, a hole-transport layer 42, a first light-emitting

layer 43, a second light-emitting layer 44, and an electron-transport layer 45.

The second electrode 5 may be a transparent electrode, a reflective electrode, or a semitransparent electrode.

The protective layer 6 reduces the penetration of moisture into the organic compound layers. The protective layer is illustrated as a single layer but may be a plurality of layers. The protective layer may include an inorganic compound layer and an organic compound layer.

The color filter 7 is divided into 7R, 7G, and 7B according to the color. The color filter may be formed on a planarizing film (not shown). Furthermore, a resin protective layer (not shown) may be provided on the color filter. The color filter may be formed on the protective layer 6. Alternatively, the color filter may be bonded after being provided on an opposite substrate, such as a glass substrate.

A display apparatus 100 in Fig. 2B includes a substrate 11 made of glass, silicon, or the like and an insulating layer 12 on the substrate 11. The display apparatus 100 includes, on the insulating layer, an active element 18, such as a TFT, and a gate electrode 13, a gate-insulating film 14, and a semiconductor layer 15 of the active element. The TFT 18 is also composed of the semiconductor layer 15, a drain electrode 16, and a source electrode 17. The TFT 18 is covered with an insulating film 19. A positive electrode 21 of the organic light-emitting element is coupled to the source electrode 17 through a contact hole 20 formed in the insulating film.

Electrical connection between electrodes of the organic light-emitting element (the positive electrode and a negative electrode) and the electrodes of the TFT (the source electrode and the drain electrode) is not limited to that illustrated in Fig. 2B. More specifically, it is only necessary to electrically connect one of the positive electrode and the negative electrode to one of the source electrode and the drain electrode of the TFT. TFT refers to a thin-film transistor.

Although an organic compound layer is a single layer in the display apparatus 100 illustrated in Fig. 2B, an organic compound layer 22 may be composed of a plurality of layers. A first protective layer 24 and a second protective layer 25 for reducing degradation of the organic light-emitting element are provided on a negative electrode 23.

Although the display apparatus 100 in Fig. 2B includes a transistor as a switching element, another switching element may be used instead.

The transistor used in the display apparatus 100 in Fig. 2B is not limited to a transistor including a single crystal silicon wafer and may also be a thin-film transistor including an active layer on an insulating surface of a substrate. The active layer may be single-crystal silicon, non-single-crystal silicon, such as amorphous silicon or microcrystalline silicon, or a non-single-crystal oxide semiconductor, such as indium zinc oxide or indium gallium zinc oxide. The thin-film transistor is also referred to as a TFT element.

The transistor in the display apparatus 100 of Fig. 2B may be formed within a substrate, such as a Si substrate. The phrase "formed within a substrate" means that the substrate, such as a Si substrate, itself is processed to form the transistor. Thus, the transistor within the substrate can be considered that the substrate and the transistor are integrally formed.

In the organic light-emitting element according to the present embodiment, the luminance is controlled with the TFT, which is an example of a switching element. The organic light-emitting element can be provided on a plurality of planes to display an image at each luminance. The switching element according to the present embodiment is not limited to the TFT and may be a transistor formed of low-temperature polysilicon or an active-matrix driver formed on a substrate, such as a Si substrate. "On a substrate" may also be referred to as "within a substrate". Whether a transistor is provided within a substrate or a TFT is used depends on the size of a display unit. For example, for an approximately 0.5-inch display unit, an organic light-emitting element is preferably provided on a Si substrate.

Fig. 3 is a schematic view of an example of the display apparatus according to the present embodiment. A display apparatus 1000 may include a touch panel 1003, a display panel 1005, a frame 1006, a circuit substrate 1007, and a battery 1008 between an upper cover 1001 and a lower cover 1009. The touch panel 1003 and the display panel 1005 are coupled to flexible print circuits FPC 1002 and 1004. Transistors are printed on the circuit substrate 1007. The battery 1008 may not be provided when the display apparatus is not a mobile device, or may be provided at another position even when the display apparatus is a mobile device.

The display apparatus according to the present embodiment may include color filters of red, green, and blue colors. The color filters may be arranged such that the red, green, and blue colors are arranged in a delta arrangement.

The display apparatus according to the present embodiment may be used for a display unit of a mobile terminal. Such a display apparatus may have both a display function and an operation function. Examples of the mobile terminal include mobile phones, such as smartphones, tablets, and head-mounted displays.

The display apparatus according to the present embodiment may be used for a display unit of an imaging apparatus that includes an optical unit with a plurality of lenses and an imaging element for receiving light passing through the optical unit. The imaging apparatus may include a display unit for displaying information acquired by the imaging element. The display unit may be a display unit exposed outside from the imaging apparatus or a display unit located in a finder. The imaging apparatus may be a digital camera or a digital video camera.

Fig. 4A is a schematic view of an example of the imaging apparatus according to the present embodiment. An imaging apparatus 1100 may include a viewfinder 1101, a rear display 1102, an operating unit 1103, and a housing

1104. The viewfinder 1101 may include the display apparatus according to the present embodiment. In such a case, the display apparatus may display environmental information, imaging instructions, and the like as well as an image to be captured. The environmental information may include the intensity of external light, the direction of external light, the travel speed of the photographic subject, and the possibility that the photographic subject is shielded by a shield.

Because the appropriate timing for imaging is a short time, it is better to display information as soon as possible. Thus, a display apparatus including an organic light-emitting element according to the present invention is preferably used. This is because the organic light-emitting element has a high response speed. A display apparatus including the organic light-emitting element can be more suitably used than these apparatuses and liquid crystal displays that require a high display speed.

The imaging apparatus 1100 includes an optical unit (not shown). The optical unit has a plurality of lenses and focuses an image on an imaging element in the housing 1104. The focus of the lenses can be adjusted by adjusting their relative positions. This operation can also be automatically performed. The imaging apparatus may also be referred to as a photoelectric conversion apparatus. The photoelectric conversion apparatus can have, as an imaging method, a method of detecting a difference from a previous image or a method of cutting out a permanently recorded image, instead of taking an image one after another.

Fig. 4B is a schematic view of an example of electronic equipment according to the present embodiment. Electronic equipment 1200 includes a display unit 1201, an operating unit 1202, and a housing 1203. The housing 1203 may include a circuit, a printed circuit board including the circuit, a battery, and a communication unit. The operating unit 1202 may be a button or a touch panel response unit. The operating unit may be a biometric recognition unit that recognizes a fingerprint and releases the lock. Electronic equipment with a communication unit may also be referred to as communication equipment. The electronic equipment may have a lens and an imaging element and thereby further have a camera function. An image captured by the camera function is displayed on the display unit. The electronic equipment may be a smartphone, a notebook computer, or the like.

Figs. 5A and 5B are schematic views of an example of the display apparatus according to the present embodiment. Fig. 5A illustrates a display apparatus, such as a television monitor or a PC monitor. A display apparatus 1300 includes a frame 1301 and a display unit 1302. The light-emitting apparatus according to the present embodiment may be used for the display unit 1302.

The frame 1301 and the display unit 1302 are supported by a base 1303. The base 1303 is not limited to the structure illustrated in Fig. 5A. The lower side of the frame 1301 may also serve as the base.

The frame 1301 and the display unit 1302 may be bent. The radius of curvature may range from 5000 to 6000 mm.

Fig. 5B is a schematic view of another example of the display apparatus according to the present embodiment. A display apparatus 1310 in Fig. 5B is configured to be foldable and is a so-called foldable display apparatus. The display apparatus 1310 includes a first display unit 1311, a second display unit 1312, a housing 1313, and a folding point 1314. The first display unit 1311 and the second display unit 1312 may include the light-emitting apparatus according to the present embodiment. The first display unit 1311 and the second display unit 1312 may be a single display apparatus without a joint. The first display unit 1311 and the second display unit 1312 can be divided by a folding point. The first display unit 1311 and the second display unit 1312 may display different images or one image.

Fig. 6A is a schematic view of an example of a lighting apparatus according to the present embodiment. A lighting apparatus 1400 may include a housing 1401, a light source 1402, a circuit board 1403, an optical film 1404, and a light diffusing unit 1405. The light source may include the organic light-emitting element according to the present embodiment. The optical filter may be a filter for improving the color rendering properties of the light source. The light diffusing unit can effectively diffuse light from the light source and widely spread light as in illumination. The optical filter and the light diffusing unit may be provided on the light output side of the illumination. If necessary, a cover may be provided on the outermost side.

For example, the lighting apparatus is an interior lighting apparatus. The lighting apparatus may emit white light, neutral white light, or light of any color from blue to red. The lighting apparatus may have a light control circuit for controlling such light. The lighting apparatus may include an organic light-emitting element according to the present invention and a power supply circuit coupled thereto. The power supply circuit is a circuit that converts an AC voltage to a DC voltage. White has a color temperature of 4200 K, and neutral white has a color temperature of 5000 K. The lighting apparatus may have a color filter.

The lighting apparatus according to the present embodiment may include a heat dissipation unit. The heat dissipation unit releases heat from the apparatus to the outside and may be a metal or liquid silicon with a high specific heat.

Fig. 6B is a schematic view of an automobile as an example of a moving body according to the present embodiment. The automobile has a taillight as an example of a lamp. An automobile 1500 may have a taillight 1501, which comes on when a brake operation or the like is performed.

The taillight 1501 may include the organic light-emitting element according to the present embodiment. The taillight may have a protective member for protecting an organic EL element. The protective member may be formed of any transparent material with moderately high strength and is preferably formed of polycarbonate or the like. The polycarbonate may be mixed with a furan dicarboxylic acid derivative, an acrylonitrile derivative, or the like.

The automobile 1500 may have a body 1503 and a window 1502 on the body 1503. The window may be a transparent display as long as it is not a window for checking the front and rear of the automobile. The transparent display may include the organic light-emitting element according to the present embodiment. In such a case, constituent materials, such as electrodes, of the organic light-emitting element are transparent materials.

The moving body according to the present embodiment may be a ship, an aircraft, a drone, or the like. The moving body may include a body and a lamp provided on the body. The lamp may emit light to indicate the position of the body. The lamp includes the organic light-emitting element according to the present embodiment.

As described above, an apparatus including the organic light-emitting element according to the present embodiment can be used to stably display a high-quality image for extended periods.

EXAMPLES

In the present exemplary embodiment, calculation is performed using a blue light-emitting material as an example, and an electron-withdrawing group and an electron-donating group are actually introduced into a device containing such a material at a specific position to improve the chromaticity of the device.

<Exemplary Embodiments 1 to 22 and Comparative Examples 1 to 11>

The emission wavelengths of exemplary compounds C1 to C25 were calculated by the following method. The following exemplary compounds C1 to C25 have mother skeletons E11, F11, and G11.

<Calculation Method of Emission Wavelength>

A transition wavelength from the ground state to an excited state calculated using the time-dependent density-functional theory (TD-B3LYP/6-31G*) for the most stable structure calculated using B3LYP/6-31G* is used as a calculation wavelength.

<Calculation Method of NBO Charge>

The natural bond orbital (NBO) charge of each atom is calculated as described below. The natural population of each atom is obtained by performing natural bond orbital (NBO) analysis [1] of an electronic state calculated by the B3LYP/6-31G* method. The NBO charge is the sum of the natural population and the atomic number corresponding to the positive charge density of each atom. The NBO charge is an indicator of the electrical polarization of each atom. (Weinhold, F.; Landis, C. R. (2001). "Natural bond orbitals and extensions of localized bonding concepts". Chem. Educ. Res. Pract. Eur. 2: 91-104.)

The molecular orbital calculation was performed using widely used Gaussian 09 (Gaussian 09, Revision C. 01, M. J. Frisch, G. W. Trucks, H. B. Schlegel, G. E. Scuseria, M. A. Robb, J. R. Cheeseman, G. Scalmani, V. Barone, B. Mennucci, G. A. Petersson, H. Nakatsuji, M. Caricato, X. Li, H. P. Hratchian, A. F. Izmaylov, J. Bloino, G. Zheng, J. L. Sonnenberg, M. Hada, M. Ehara, K. Toyota, R. Fukuda, J. Hasegawa, M. Ishida, T. Nakajima, Y. Honda, O. Kitao, H. Nakai, T. Vreven, J. A. Montgomery, Jr., J. E. Peralta, F. Ogliaro, M. Bearpark, J. J. Heyd, E. Brothers, K. N. Kudin, V. N. Staroverov, T. Keith, R. Kobayashi, J. Normand, K. Raghavachari, A. Rendell, J. C. Burant, S. S. Iyengar, J. Tomasi, M. Cossi, N. Rega, J. M. Millam, M. Klene, J. E. Knox, J. B. Cross, V. Bakken, C. Adamo, J. Jaramillo, R. Gomperts, R. E. Stratmann, O. Yazyev, A. J. Austin, R. Cammi, C. Pomelli, J. W. Ochterski, R. L. Martin, K. Morokuma, V. G. Zakrzewski, G. A. Voth, P. Salvador, J. J. Dannenberg, S. Dapprich, A. D. Daniels, O. Farkas, J. B. Foresman, J. V. Ortiz, J. Cioslowski, and D. J. Fox, Gaussian, Inc., Wallingford CT, 2010.).

[Chem. 12]

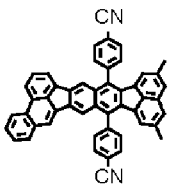
C1

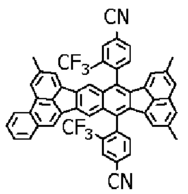
C2

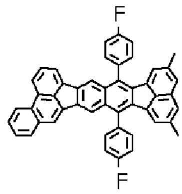
C3

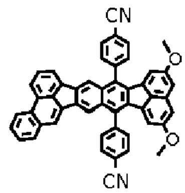
C4

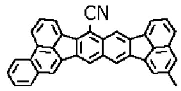
C5

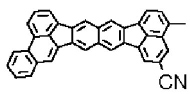
C6

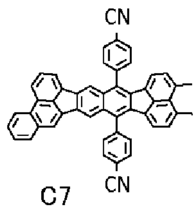
C7

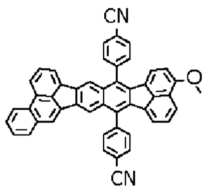
C8

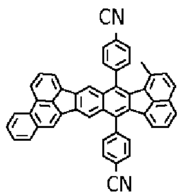
C9

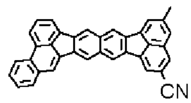
C10

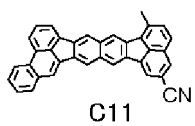
C11

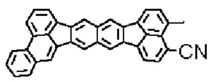
C12

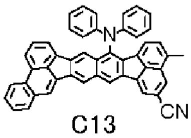
C13

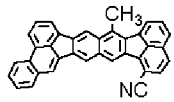
C14

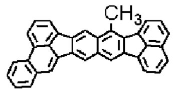
C15

[Chem. 13]

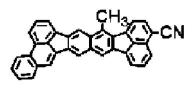

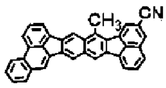

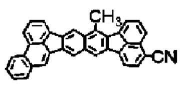

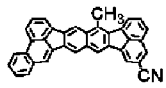

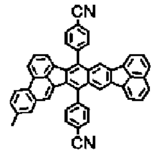

C16 C17 C18 C19 C20

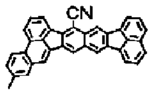

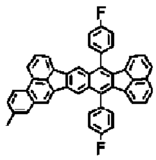

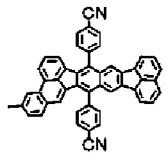

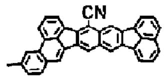

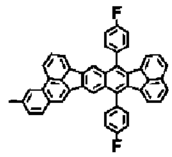

C21 C22 C23 C24 C25

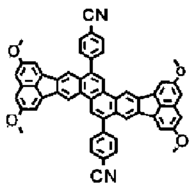

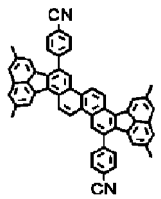

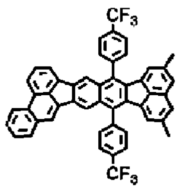

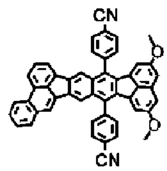

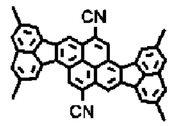

C26 C27 C28 C29 C30

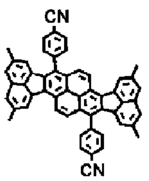

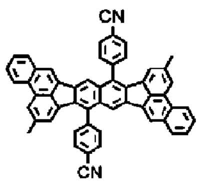

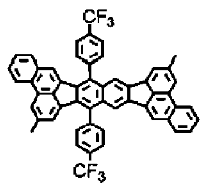

C31 C32 C33

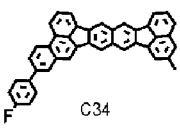

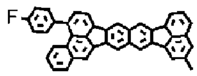

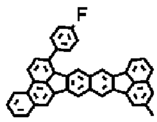

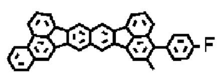

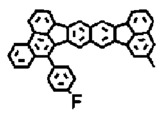

C34 C35 C36 C37 C38

An exemplary compound C group can be synthesized by the following synthetic route, for example.

<Synthesis Examples>

Synthesis examples of the exemplary compounds C3 and C25 as examples of organic compounds represented by the general formulae E11, F11, and G11 are described below.

[Synthesis Example 1] Synthesis of Exemplary Compound C3

[Chem. 14]

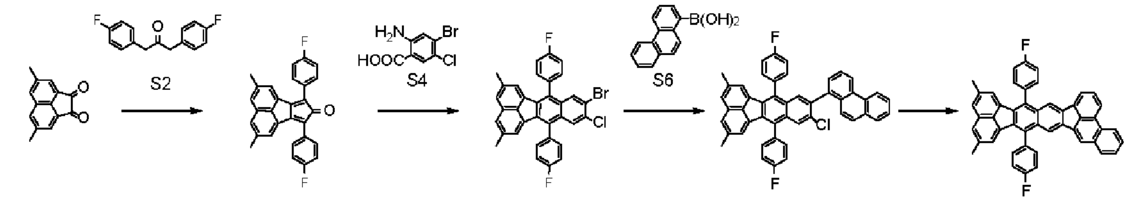


S1 S3 S5 S7 C3

(1) Synthesis of Compound S3

A 200-ml recovery flask was charged with the following reagents and solvent.

Compound E1: 2.10 g (10 mmol)
Compound E2: 2.46 g (10 mmol)
Ethanol: 100 ml

Next, the reaction solution was heated to 70°C in a nitrogen stream, and a KOH ethanol solution was added dropwise to the reaction solution. The reaction solution was stirred at this temperature (70°C) for 6 hours. After completion of the reaction, water was added to the product, and the precipitate was filtered. The filter cake was dispersed and washed with methanol. Thus, 3.15 g (yield: 75%) of a dark gray compound S3 was produced.

(2) Synthesis of Compound S5

A 100-ml recovery flask was charged with the following reagents and solvent.

Compound S3: 2.94 g (7 mmol)
Compound S4: 2.25 g (9 mmol)
Isoamyl nitrite: 1.05 g (9 mmol)
Toluene: 40 ml

Next, the reaction solution was heated to 110°C in a nitrogen stream and was stirred at this temperature (80°C) for 3 hours. After completion of the reaction, the product was washed twice with 40 ml of water. The organic layer was washed with saturated saline, was dried over magnesium sulfate, and was filtered. The filtrate was concentrated. A brown liquid was produced. The brown liquid was purified by column chromatography (chloroform/heptane = 1:4) and was then recrystallized in chloroform/methanol. Thus, 3.46 g (yield: 85%) of a yellow crystalline S5 was produced.

(3) Synthesis of Compound S7

A 200-ml recovery flask was charged with the following reagents and solvents.

Compound S5: 1.75 g (3 mmol)
Compound S6: 0.67 g (3 mmol)
$Pd(PPh_3)_4$: 0.2 g
Toluene: 50 ml
Ethanol: 20 ml
2M aqueous sodium carbonate: 50 ml

Next, the reaction solution was heated to 80°C in a nitrogen stream and was stirred at this temperature (80°C) for 6 hours. After completion of the reaction, water was added to the product for separation. The product was dissolved in chloroform, was purified by column chromatography (chloroform), and was recrystallized in chloroform/methanol. Thus, 1.53 g (yield: 75%) of a yellow crystalline compound S7 was produced.

(4) Synthesis of Exemplary Compound C3

A 20-ml recovery flask was charged with the following reagents and solvent.

Compound S7: 680 mg (1 mmol)
Pd(dba)2: 238 mg
P(Cy)3 (tricyclohexylphosphine): 280 mg
DBU (diazabicycloundecene): 0.15 ml
DMF: 5 ml

Next, the reaction solution was heated to 145°C in a nitrogen stream and was stirred at this temperature (145°C) for 6 hours. After completion of the reaction, ethanol was added to precipitate crystals. The crystals were separated by filtration and were dispersed and washed successively with water, ethanol, and heptane. The resulting purple crystals were then heated and dissolved in toluene, were subjected to hot filtration, and were recrystallized in toluene/methanol.

Thus, 0.50 g (yield: 78%) of a dark red exemplary compound C3 was produced.

The compound had a purity of 99% or more as measured by HPLC.

The exemplary compound C3 was subjected to mass spectrometry with MALDI-TOF-MS (Autoflex LRF manufactured by Bruker).

[MALDI-TOF-MS]

Actual value: m/z = 642.85 Calculated value: C46H20N4 = 642.75

[Synthesis Example 2] Synthesis of Exemplary Compound C25

[Chem. 15]

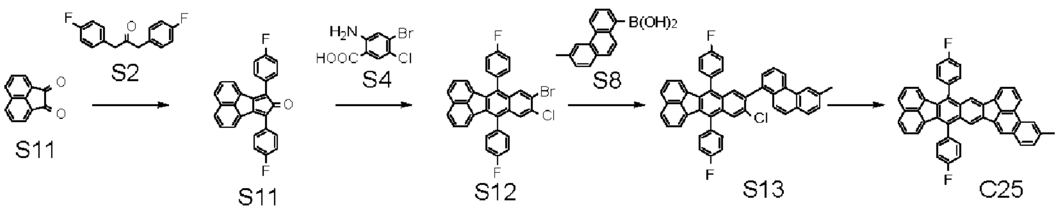


(1) Synthesis of Compound S11

A 200-ml recovery flask was charged with the following reagents and solvent.

Compound S11: 1.82 g (10 mmol)
Compound S2: 2.46 g (10 mmol)
Ethanol: 100 ml

Next, the reaction solution was heated to 70°C in a nitrogen stream, and a KOH ethanol solution was added dropwise to the reaction solution. The reaction solution was stirred at this temperature (70°C) for 6 hours. After completion of the reaction, water was added to the product, and the precipitate was filtered. The filter cake was dispersed and washed with methanol. Thus, 2.94 g (yield: 75%) of a dark gray compound S11 was produced.

(2) Synthesis of Compound S5

A 100-ml recovery flask was charged with the following reagents and solvent.

Compound S11: 2.75 g (7 mmol)
Compound S4: 2.25 g (9 mmol)
Isoamyl nitrite: 1.05 g (9 mmol)
Toluene: 40 ml

Next, the reaction solution was heated to 110°C in a nitrogen stream and was stirred at this temperature (80°C) for 3 hours. After completion of the reaction, the product was washed twice with 40 ml of water. The organic layer was washed with saturated saline, was dried over magnesium sulfate, and was filtered. The filtrate was concentrated. A brown liquid was produced. The brown liquid was purified by column chromatography (chloroform/heptane = 1:4) and was then recrystallized in chloroform/methanol. Thus, 3.30 g (yield: 85%) of a yellow crystalline E5 was produced.

(3) Synthesis of Compound S7

A 200-ml recovery flask was charged with the following reagents and solvents.

Compound S12: 1.66 g (3 mmol)
Compound S8: 0.71 g (3 mmol)

$Pd(PPh_3)_4$: 0.2 g
Toluene: 50 ml
Ethanol: 20 ml
2M aqueous sodium carbonate: 50 ml

Next, the reaction solution was heated to 80°C in a nitrogen stream and was stirred at this temperature (80°C) for 6 hours. After completion of the reaction, water was added to the product for separation. The product was dissolved in chloroform, was purified by column chromatography (chloroform), and was recrystallized in chloroform/methanol. Thus, 1.50 g (yield: 75%) of a yellow crystalline compound S7 was produced.

(4) Synthesis of Exemplary Compound C3

A 20-ml recovery flask was charged with the following reagents and solvents.

Compound S7: 670 mg (1 mmol)
Pd(dba)2: 238 mg
P(Cy)3 (tricyclohexylphosphine): 280 mg
DBU (diazabicycloundecene): 0.15 ml
DMF: 5 ml

Next, the reaction solution was heated to 145°C in a nitrogen stream and was stirred at this temperature (145°C) for 6 hours. After completion of the reaction, ethanol was added to precipitate crystals. The crystals were separated by filtration and were dispersed and washed successively with water, ethanol, and heptane. The resulting purple crystals were then heated and dissolved in toluene, were subjected to hot filtration, and were recrystallized in toluene/methanol. Thus, 0.49 g (yield: 78%) of a dark red exemplary compound C3 was produced.
The compound had a purity of 99% or more as measured by HPLC.
The exemplary compound C3 was subjected to mass spectrometry with MALDI-TOF-MS (Autoflex LRF manufactured by Bruker).

[MALDI-TOF-MS]

Actual value: m/z = 628.80 Calculated value: $C_{46}H_{20}N_4$ = 628.72
Table 3 shows the calculation results. The calculated short-wavelength shift values in Table 3 are values obtained by subtracting the Stokes shift. This is because the calculation includes the Stokes shift. When the calculated short-wavelength shift value is 0, a calculated shift value resulting from the Stokes shift alone is calculated.
In Table 3, the condition 1 is "The electric charge value of the natural bond orbital (NBO) of the carbon atom bonded to the electron-donating group is equal to or lower than the electric charge values of the natural bond orbitals of carbon atoms on both sides of the carbon atom." described in (2)-2. The condition 2 is "The electric charge value of the natural bond orbital (NBO) of the carbon atom bonded to the electron-donating group is lower by 0.02 or more than one of the NBO charge values of carbon atoms on both sides of the carbon atom." described in (2)-3.

[Table 3]

Table 3

| Exemplary embodiment No. | Compound name | $\Sigma\|C_H\|>\Sigma\|C_L\|$ | Electron-withdrawing group E-Eb (eV) | Condition 1 | Condition 2 | Calculated short-wavelength shift (nm) | Wavelength shortening effect |
|---|---|---|---|---|---|---|---|
| Exemplary embodiment 1 | C1 | ○ | -1.77 | ○ | ○ | 4.5 | ○ |
| Exemplary embodiment 2 | C2 | ○ | -2.04 | ○ | ○ | 6.4 | ○ |

(continued)

| Exemplary embodiment No. | Compound name | $\Sigma\|C_H\|>\Sigma\|C_L\|$ | Electron-withdrawing group E-Eb (eV) | Condition 1 | Condition 2 | Calculated short-wavelength shift (nm) | Wavelength shortening effect |
|---|---|---|---|---|---|---|---|
| Exemplary embodiment 3 | C3 | ○ | -0.81 | ○ | ○ | 1 | ○ |
| Exemplary embodiment 4 | C4 | ○ | -1.77 | ○ | ○ | 4.4 | ○ |
| Exemplary embodiment 5 | C5 | ○ | -1.51 | ○ | ○ | 4.9 | ○ |
| Exemplary embodiment 6 | C20 | ○ | -1.77 | ○ | × | 1.3 | ○ |
| Exemplary embodiment 7 | C21 | ○ | -1.51 | ○ | × | 4.2 | ○ |
| Exemplary embodiment 8 | C23 | ○ | -1.77 | ○ | × | 0.1 | ○ |
| Exemplary embodiment 9 | C24 | ○ | -1.51 | ○ | × | 2.7 | ○ |
| Exemplary embodiment 10 | C26 | ○ | -1.77 | ○ | ○ | 1 | ○ |
| Exemplary embodiment 11 | C27 | ○ | -1.77 | ○ | ○ | 8.5 | ○ |
| Exemplary embodiment 12 | C28 | ○ | -1.3 | ○ | ○ | 2.7 | ○ |
| Exemplary embodiment 13 | C29 | ○ | -1.77 | ○ | ○ | 5.2 | ○ |
| Exemplary embodiment 14 | C30 | ○ | -1.51 | ○ | ○ | 13.8 | ○ |
| Exemplary embodiment 15 | C31 | ○ | -1.77 | ○ | ○ | 6.6 | ○ |
| Exemplary embodiment 16 | C32 | ○ | -1.77 | ○ | ○ | 3.3 | ○ |
| Exemplary embodiment 17 | C33 | ○ | -1.3 | ○ | ○ | 2.3 | ○ |

(continued)

| Exemplary embodiment No. | Compound name | $\Sigma\|C_H\|>\Sigma\|C_L\|$ | Electron-withdrawing group E-Eb (eV) | Condition 1 | Condition 2 | Calculated short-wavelength shift (nm) | Wavelength shortening effect |
|---|---|---|---|---|---|---|---|
| Exemplary embodiment 18 | C7 | ○ | -1.77 | × | × | -2 | Δ |
| Exemplary embodiment 19 | C8 | ○ | -1.77 | × | × | -0.4 | Δ |
| Exemplary embodiment 20 | C9 | ○ | -1.77 | × | × | -5.6 | Δ |
| Exemplary embodiment 21 | C22 | ○ | -0.81 | ○ | × | -1.5 | Δ |
| Exemplary embodiment 22 | C25 | ○ | -0.81 | ○ | × | -2.3 | Δ |
| Comparative example 1 | C6 | × | -1.51 | × | × | -14.5 | × |
| Comparative example 2 | C10 | × | -1.51 | ○ | ○ | -9.2 | × |
| Comparative example 3 | C11 | × | -1.51 | × | × | -12.7 | × |
| Comparative example 4 | C12 | × | -1.51 | × | × | -31.4 | × |
| Comparative example 5 | C13 | × | -1.51 | × | × | -17 | × |
| Comparative example 6 | C14 | × | -1.51 | ○ | ○ | -34.1 | × |
| Comparative example 7 | C15 | | - | ○ | ○ | -4.4 | × |
| Comparative example 8 | C16 | × | -1.51 | ○ | ○ | -33 | × |
| Comparative example 9 | C17 | × | -1.51 | ○ | ○ | -17.2 | × |
| Comparative example 10 | C18 | × | -1.51 | ○ | ○ | -29.9 | × |
| Comparative example 11 | C19 | × | -1.51 | ○ | ○ | -15.5 | × |

<Exemplary Embodiments 1 to 5>

The organic compounds of Exemplary Embodiments 1 to 5 are organic compounds represented by the general formula G11. In these organic compounds, the substituent position of the electron-withdrawing group satisfies the formula (1), and the substitution position of the electron-donating group satisfies (2)-1 to (2)-3. In Exemplary Embodiments 1 to 5, the emission wavelength of the organic compound is shortened due to the effects of the electron-withdrawing group

and the electron-donating group.

<Exemplary Embodiments 6 to 9>

The organic compounds of Exemplary Embodiments 6 to 9 are organic compounds represented by the general formula E1. In these organic compounds, the substitution position of the electron-withdrawing group satisfies the formula (1). The organic compound represented by the general formula E1 has a shorter wavelength due to the effect of the electron-withdrawing group.

<Exemplary Embodiments 10 to 17>

In the organic compounds of Exemplary Embodiments 10 to 17, the substitution position of the electron-withdrawing group satisfies the formula (1), and the substitution position of the electron-donating group satisfies the formula (2). The wavelength is also shortened in the compounds belonging to the general formulae other than the organic compounds described in Exemplary Embodiments 1 to 9.

<Exemplary Embodiments 18 to 22>

Although the organic compounds of Exemplary Embodiments 18 to 22 satisfy the condition of the formula (1), an electron-donating group is provided in addition to the electron-withdrawing group. Because the substitution position of the electron-withdrawing group does not satisfy (2), the whole compound has a longer wavelength than the mother skeleton. However, the wavelength is shortened as compared with the compounds without the electron-withdrawing group, and the rating is Δ between O and X. Although the electron-withdrawing group has the effect of shortening the wavelength, satisfying the condition of the electron-donating group is more preferred.

<Exemplary Embodiment 23>

As shown in Fig. 1D, the electron-withdrawing group of the compound C34 in Exemplary Embodiment 23 substitutes for at a carbon of an aromatic ring at an end of a mother skeleton without the LUMO orbital coefficient. The wavelength of C34 is shortened by satisfying (2).

[Table 4]

Table 4

| Exemplary embodiment No. | Compound name | $C_L$-0 | Condition 2 | Calculated short-wavelength shift (nm) | Shortening of wavelength |
|---|---|---|---|---|---|
| Exemplary embodiment 23 | C34 | ○ | ○ | 0.2 | ○ |
| Comparative example 12 | C35 | x | ○ | -2.7 | x |
| Comparative example 13 | C36 | x | ○ | -5.1 | x |
| Comparative example 14 | C37 | x | ○ | -5.1 | x |
| Comparative example 15 | C38 | x | ○ | -2.2 | x |

<Exemplary Embodiment 24>

In the present exemplary embodiment, an organic light-emitting element with the structure shown in Table 5 was produced, as described below. More specifically, it is an organic light-emitting element of a bottom emission type including a positive electrode, a hole-injection layer, a hole-transport layer, an electron-blocking layer, a light-emitting

layer, a hole-blocking layer, an electron-transport layer, an electron-injection layer, and a negative electrode on a substrate.

First, an ITO film was formed on a glass substrate and was subjected to desired patterning to form an ITO electrode (positive electrode). The ITO electrode had a thickness of 100 nm. The substrate on which the ITO electrode was formed was used as an ITO substrate in the following process. Vacuum evaporation was then performed by resistance heating in a vacuum chamber at 1.33 x $10^{-4}$ Pa to continuously form an organic compound layer and an electrode layer shown in Table 5 on the ITO substrate. The counter electrode (a metal electrode layer, a negative electrode) had an electrode area of 3 $mm^2$.

[Table 5]

Table 5

| | Material | | | Thickness/ nm |
|---|---|---|---|---|
| Negative electrode | Al | | | 100 |
| Electron-injection layer (EIL) | LiF | | | 1 |
| Electron-transport layer (ETL) | ET5 | | | 35 |
| Hole-blocking layer (HBL) | ET17 | | | 10 |
| Light-emitting layer (EML) | Host | EM3 | Weight ratio EM3:C1 = 99:1 | 25 |
| | Guest | C1 | | |
| Electron-blocking layer (EBL) | HT12 | | | 45 |
| Hole-transport layer (HTL) | HT3 | | | 68 |
| Hole-injection layer (HIL) | HT16 | | | 5 |

<Exemplary Embodiments 25 to 32, Comparative Examples 17 to 32>

An organic light-emitting element was produced in the same manner as in Exemplary Embodiment 24 except that the guest in the light-emitting layer was changed to a compound shown in Table 6. The exemplary compound A41 and the exemplary compound C1, the exemplary compound A42 and the exemplary compound C3, and the exemplary compound B24 and the exemplary compound C4 are the same compound.

<Improvement of Chromaticity Coordinates of Organic Light-Emitting Element>

The effect of shortening the wavelength of an organic light-emitting element was evaluated as the chromaticity coordinates of an emission spectrum of the organic light-emitting element. An emission spectrum of a produced organic light-emitting element was measured. The current-voltage characteristics were measured with a microammeter 4140B manufactured by Hewlett-Packard Co., and the luminance and emission spectrum were measured with SR-3A manufactured by Topcon Corporation. An emission spectrum was measured at an electric current of 10 $mA/cm^2$.

The chromaticity of an emission spectrum of an organic light-emitting element was compared with that of a device produced using an unsubstituted mother skeleton as a light-emitting material. Table 6 shows the results together with the results of Exemplary Embodiment 24. The light-emitting materials in the present exemplary embodiments emit blue light. Thus, a change in chromaticity coordinates in the direction of increasing the purity of the blue light emission was judged to be "effective (O)". For example, Exemplary Embodiment 24 is an organic light-emitting element produced using the exemplary compound C1, and exhibited improvement in the chromaticity of blue light emission with respect to an organic light-emitting element produced using an unsubstituted mother skeleton (not described).

Consider CIE(x, y) = (0.15, 0.06) of the sRGB standard as a reference value for the target chromaticity of blue light emission. The emission chromaticity of the organic light-emitting element of each exemplary embodiment shifted toward the target chromaticity coordinates (0.15, 0.06), and the chromaticity coordinates were improved.

<Drive Life of Organic Light-Emitting Element>

A produced organic light-emitting element was subjected to a continuous operation test at a current density of

100 mA/$cm^2$ to measure the half-life of luminance degradation. The criterion was 180 hours or more for A and less than 180 hours for B. Table 6 shows the results.

[Table 6]

Table 6

| Exemplary embodiment No. | EML | | Improvement in chromaticity coordinates | Improvement in drive half-life |
|---|---|---|---|---|
| | Host | Guest | | |
| Exemplary embodiment 24 | EM3 | C1 | ○ | A |
| Exemplary embodiment 25 | EM3 | C2 | ○ | A |
| Exemplary embodiment 26 | EM3 | C3 | ○ | A |
| Exemplary embodiment 27 | EM3 | C4 | ○ | B |
| Exemplary embodiment 28 | EM3 | C5 | ○ | A |
| Exemplary embodiment 29 | EM3 | C20 | ○ | A |
| Exemplary embodiment 30 | EM3 | C21 | ○ | A |
| Exemplary embodiment 31 | EM3 | C23 | ○ | A |
| Exemplary embodiment 32 | EM3 | C24 | ○ | A |
| Comparative example 16 | EM3 | C6 | × | A |
| Comparative example 17 | EM3 | C7 | × | A |
| Comparative example 18 | EM3 | C8 | × | B |
| Comparative example 19 | EM3 | C9 | × | A |
| Comparative example 20 | EM3 | C10 | × | A |
| Comparative example 21 | EM3 | C11 | × | A |
| Comparative example 22 | EM3 | C12 | × | A |
| Comparative example 23 | EM3 | C13 | × | B |
| Comparative example 24 | EM3 | C14 | × | A |
| Comparative example 25 | EM3 | C15 | × | A |
| Comparative example 26 | EM3 | C16 | × | A |
| Comparative example 27 | EM3 | C17 | × | A |
| Comparative example 28 | EM3 | C18 | × | A |
| Comparative example 29 | EM3 | C19 | × | A |
| Comparative example 30 | EM3 | C22 | × | A |
| Comparative example 31 | EM3 | C25 | × | A |

Table 6 shows that Exemplary Embodiments 24 to 26 and Exemplary Embodiments 28 to 32 had a half-life of 180 hours or more. By contrast, Exemplary Embodiment 21 had a half-life of less than 180 hours. Comparative Examples 18 and 23 had a shorter drive life than other comparative examples and were rated B. The organic compounds in these exemplary embodiments and comparative examples did not satisfy the condition (3) or (4) in the present description and had relatively low stability, thus resulting in a relatively short drive life.

The results of Exemplary Embodiments 24 to 32 show that an organic light-emitting element with improved chromaticity of blue light emission and with long drive life can be provided.

<Exemplary Embodiment 33>

In the present exemplary embodiment, an organic light-emitting element with the structure shown in Table 7 was produced. More specifically, it is an organic light-emitting element of a top emission type including a positive electrode, a hole-injection layer, a hole-transport layer, an electron-blocking layer, a first light-emitting layer, a second light-emitting layer, a hole-blocking layer, an electron-transport layer, an electron-injection layer, and a negative electrode on a substrate.

A 40-nm multilayer film of Al and Ti was formed on a glass substrate by a sputtering method and was patterned by photolithography to form a positive electrode. The counter electrode (a metal electrode layer, a negative electrode) had an electrode area of 3 $mm^2$. Subsequently, the substrate on which up to a cleaned electrode was formed and a material were mounted in a vacuum evaporator (manufactured by ULVAC, Inc.). After the vacuum evaporator was evacuated to 1.33 x $10^{-4}$ Pa (1 x $10^{-6}$ Torr), UV/ozone cleaning was performed. Subsequently, a film with a layer structure shown in Table 7 was formed and was finally sealed in a nitrogen atmosphere.

[Table 7]

Table 7

| | Material | | | Thickness (nm) |
|---|---|---|---|---|
| Negative electrode | Mg | Weight ratio Mg:Ag =50:50 | | 10 |
| | Ag | | | |
| Electron-injection layer (EIL) | LiF | | | 1 |
| Electron-transport layer (ETL) | ET2 | | | 30 |
| Hole-blocking layer (HBL) | ET12 | | | 70 |
| 2nd light-emitting layer (2nd EML) | Second host | EM1 | Weight ratio EM1:C1=99.4:0.6 | 10 |
| | Second guest (blue dopant) | A1 | | |
| 1st light-emitting layer (1st EML) | First host | EM1 | Weight ratio EM1:RD7:GD9=96.7: 0.3:3.0 | 10 |
| | First guest (red dopant) | RD7 | | |
| | Third guest (green dopant) | GD9 | | |
| Electron-blocking layer (EBL) | HT7 | | | 10 |
| Hole-transport layer (HTL) | HT2 | | | 20 |
| Hole-injection layer (HIL) | HT16 | | | 5 |

The characteristics of the element were measured and evaluated. The element emitted good white light. The chromaticity coordinates of blue after transmission through an RGB color filter were estimated from the resulting white emission spectrum, and the chromaticity coordinates of blue in sRGB were (0.15, 0.12).

<Exemplary Embodiments 34 to 36, Comparative Examples 32 to 34>

Organic light-emitting elements were produced in the same manner as in Exemplary Embodiment 33 except that the compounds shown in Table 8 were used. The characteristics of the elements were measured and evaluated in the same manner as in Exemplary Embodiment 33. The criterion was 180 hours or more for A and less than 180 hours for B. Table 8 shows the measurement results.

[Table 8]

Table 8

| Exemplary embodiment No. | 1st EML | | | 2nd EML | | Blue chromaticity coordinates | Drive half-life |
|---|---|---|---|---|---|---|---|
| | First host | First guest | Third guest | Second host | Second guest | (x,y) | |
| Exemplary embodiment 33 | EM1 | RD7 | GD9 | EM1 | C1 | (0.15,0.12) | A |
| Exemplary embodiment 34 | EM1 | RD7 | GD9 | EM1 | C3 | (0.15,0.12) | A |
| Exemplary embodiment 35 | EM1 | RD7 | GD9 | EM1 | C4 | (0.15,0.13) | B |
| Com parative example 32 | EM1 | RD7 | GD9 | EM1 | C7 | (0.16,0.19) | A |
| Com parative example 33 | EM1 | RD7 | GD9 | EM1 | C8 | (0.18,0.27) | B |
| Com parative example 34 | EM1 | RD7 | GD9 | EM1 | C13 | (0.19,0.27) | B |

Table 8 shows that the blue chromaticity coordinates were improved and were closer to pure blue in Exemplary Embodiments 33 to 35. The blue chromaticity coordinates were improved compared with those of devices produced using an unsubstituted mother skeleton as a light-emitting material. By contrast, the blue chromaticity coordinates were distant from pure blue in Comparative Examples 32 to 34. Exemplary Embodiments 33 and 34, in which the mother skeleton and the substituent were via a carbon-carbon bond, had longer drive half-life than Exemplary Embodiment 35.

These results show that when compared as white-light-emitting elements, white-light-emitting elements of the present invention can expand the color reproduction range with respect to the color reproduction range of sRGB in a blue light emission region. This is because an organic compound according to an embodiment of the present invention emits blue light at a shorter wavelength.

The present invention is not limited to these embodiments, and various changes and modifications may be made therein without departing from the spirit and scope of the present invention. Thus, the following claims are attached to make the scope of the present invention public.

This application claims the benefit of Japanese Patent Application No. 2019-200318 filed November 1, 2019, which is hereby incorporated by reference herein in its entirety.

## Claims

1. An organic compound comprising: a mother skeleton with a fused-ring structure; an electron-withdrawing group bonded to the mother skeleton; and an electron-donating group bonded to the mother skeleton, wherein the electron-withdrawing group is bonded at a position satisfying the following relationship in the mother skeleton.

$$\Sigma|C_H| > \Sigma|C_L| \qquad (1)$$

($C_H$: 2PZ atomic orbital coefficient of a carbon at a substitution site in HOMO of the mother skeleton)
($C_L$: 2PZ atomic orbital coefficient of the carbon at the substitution site in LUMO of the mother skeleton)

2. An organic compound, wherein the electron-withdrawing group satisfies the following formula (2), the electron-donating group is provided on a carbon atom in a mother skeleton such that a substituent can be introduced into at least one of carbon atoms on both sides of the carbon atom, and an electric charge value of a natural bond orbital of the carbon atom having the electron-donating group in the mother skeleton is equal to or lower than an electric

charge value of a natural bond orbital of one carbon atom adjacent to the carbon atom and equal to or lower than an electric charge value of a natural bond orbital of the other carbon atom adjacent to the carbon atom.

$$E - Eb \leq -1.51\ (eV) \quad (2)$$

(E: a LUMO level of a substituent in the mother skeleton, Eb: a LUMO level of a structure in which the electron-withdrawing group is removed from the substituent)

**3.** The organic compound according to Claim 2, wherein an electric charge value of a natural bond orbital of the carbon atom having the electron-donating group in the mother skeleton is lower by 0.02 or more than an electric charge value of a natural bond orbital of one of carbon atoms on both sides of the carbon atom.

**4.** An organic compound comprising: a mother skeleton with a fused-ring structure; an electron-withdrawing group bonded to the mother skeleton; and an electron-donating group bonded to the mother skeleton, wherein
the electron-donating group is provided on a carbon atom in a mother skeleton such that a substituent can be introduced into at least one of carbon atoms on both sides of the carbon atom, and an electric charge value of a natural bond orbital of the carbon atom having the electron-donating group in the mother skeleton is equal to or lower than an electric charge value of a natural bond orbital of one carbon atom adjacent to the carbon atom and equal to or lower than an electric charge value of a natural bond orbital of the other carbon atom adjacent to the carbon atom.

**5.** The organic compound according to any one of Claims 1 to 4, wherein the mother skeleton has one or more 5-membered rings.

**6.** The organic compound according to any one of Claims 1 to 5, wherein the electron-donating group is one of a methyl group, an ethyl group, a methoxy group, an ethoxy group, a t-butyl group, and aryl groups substituted therewith.

**7.** The organic compound according to any one of Claims 1 to 6, wherein the electron-withdrawing group is one of a cyano group, fluorine, chlorine, bromine, and aryl groups having these.

**8.** An organic light-emitting element comprising:

a positive electrode and a negative electrode; and
an organic compound layer between the positive electrode and the negative electrode, wherein the organic compound layer contains the organic compound according to any one of Claims 1 to 7.

**9.** The organic light-emitting element according to Claim 8, wherein the organic compound layer is a light-emitting layer.

**10.** The organic light-emitting element according to Claim 9, wherein the organic light-emitting element emits blue light.

**11.** The organic light-emitting element according to Claim 8 or 9, further comprising another light-emitting layer on the light-emitting layer, wherein the other light-emitting layer emits light of a color different from an emission color of the light-emitting layer.

**12.** The organic light-emitting element according to Claim 11, wherein the organic light-emitting element emits white light.

**13.** The organic light-emitting element according to any one of Claims 1 to 12, further comprising a color filter.

**14.** A display apparatus comprising a plurality of pixels, wherein at least one of the plurality of pixels includes the organic light-emitting element according to any one of Claims 8 to 13 and a transistor coupled to the organic light-emitting element.

**15.** A photoelectric conversion apparatus comprising:

an optical unit with a plurality of lenses; an imaging element configured to receive light passing through the optical unit; and a display unit configured to display an image taken by the imaging element,
wherein the display unit includes the organic light-emitting element according to any one of Claims 8 to 13.

**16.** Electronic equipment comprising: a display unit including the organic light-emitting element according to any one of Claims 8 to 13; a housing in which the display unit is provided; and a communication unit configured to communicate with an outside provided in the housing.

**17.** A lighting apparatus comprising: a light source including the organic light-emitting element according to any one of Claims 8 to 13; and a light-diffusing unit or an optical filter that transmits light emitted by the light source.

**18.** A moving body comprising: a lamp including the organic light-emitting element according to any one of Claims 8 to 13; and a body to which the lamp is provided.

FIG. 1A

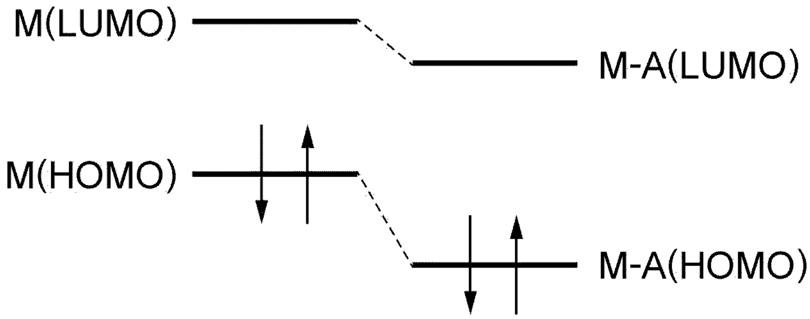


FIG. 1B

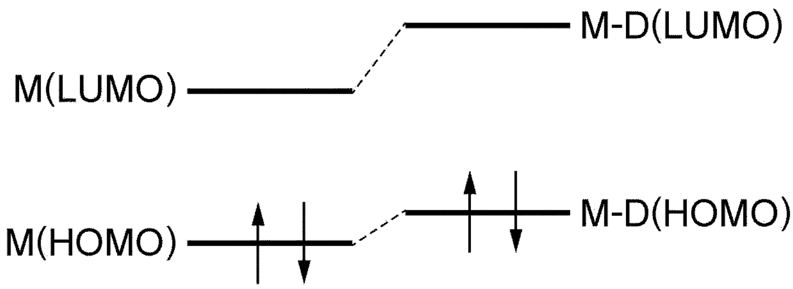


FIG. 1C

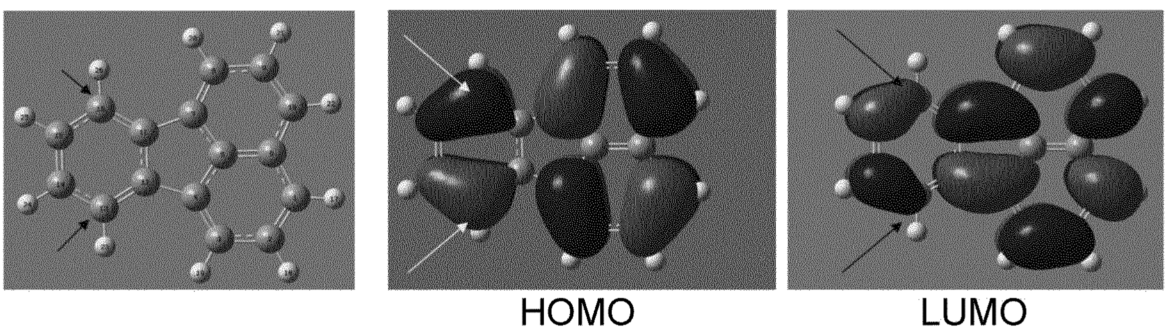

FIG. 1D

(A)

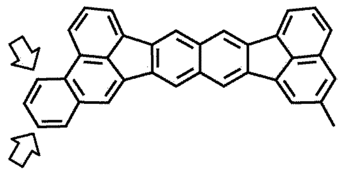

(B)

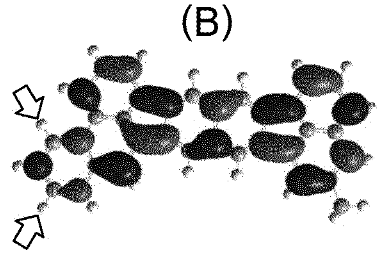

FIG. 1E

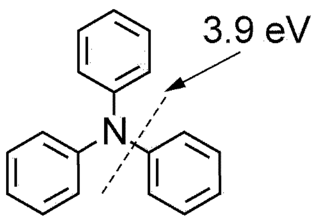


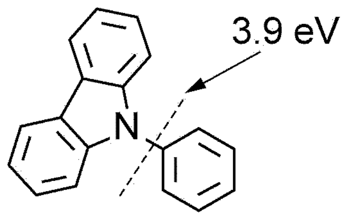


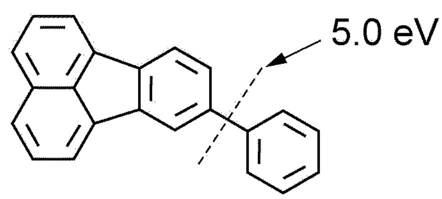

FIG. 2A

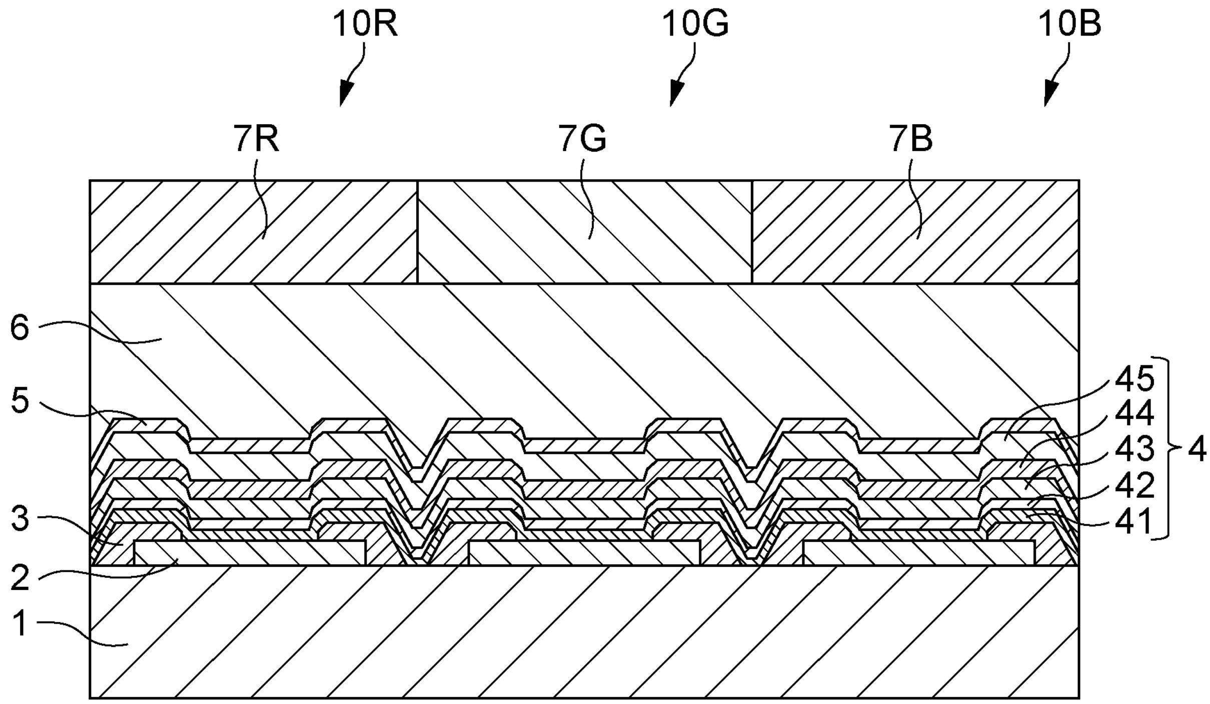
10R
10G
10B
7R
7G
7B
6
5
3
2
1
45
44
43
42
41
4

FIG. 2B

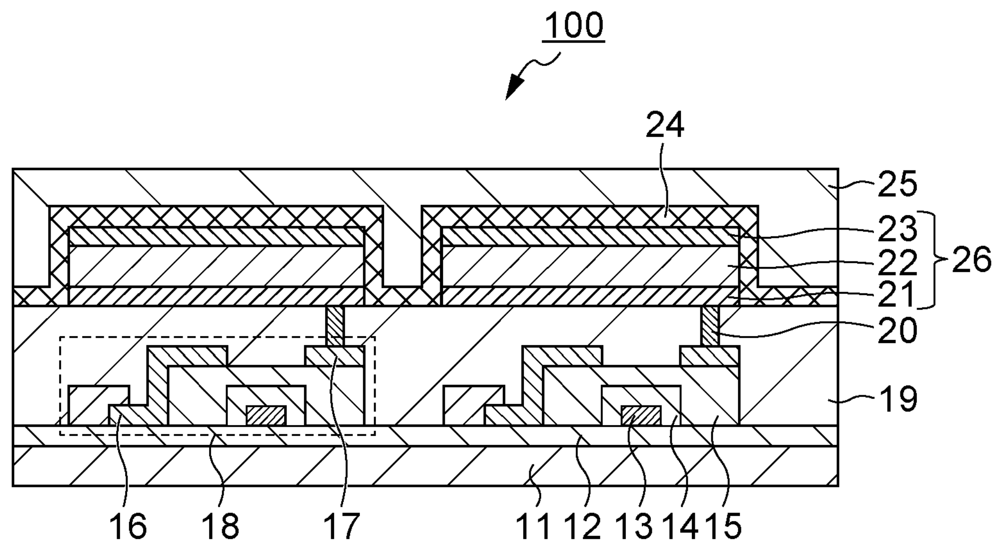
100
24
25
23
22
21
26
20
19
16
18
17
11
12
13
14
15

FIG. 3

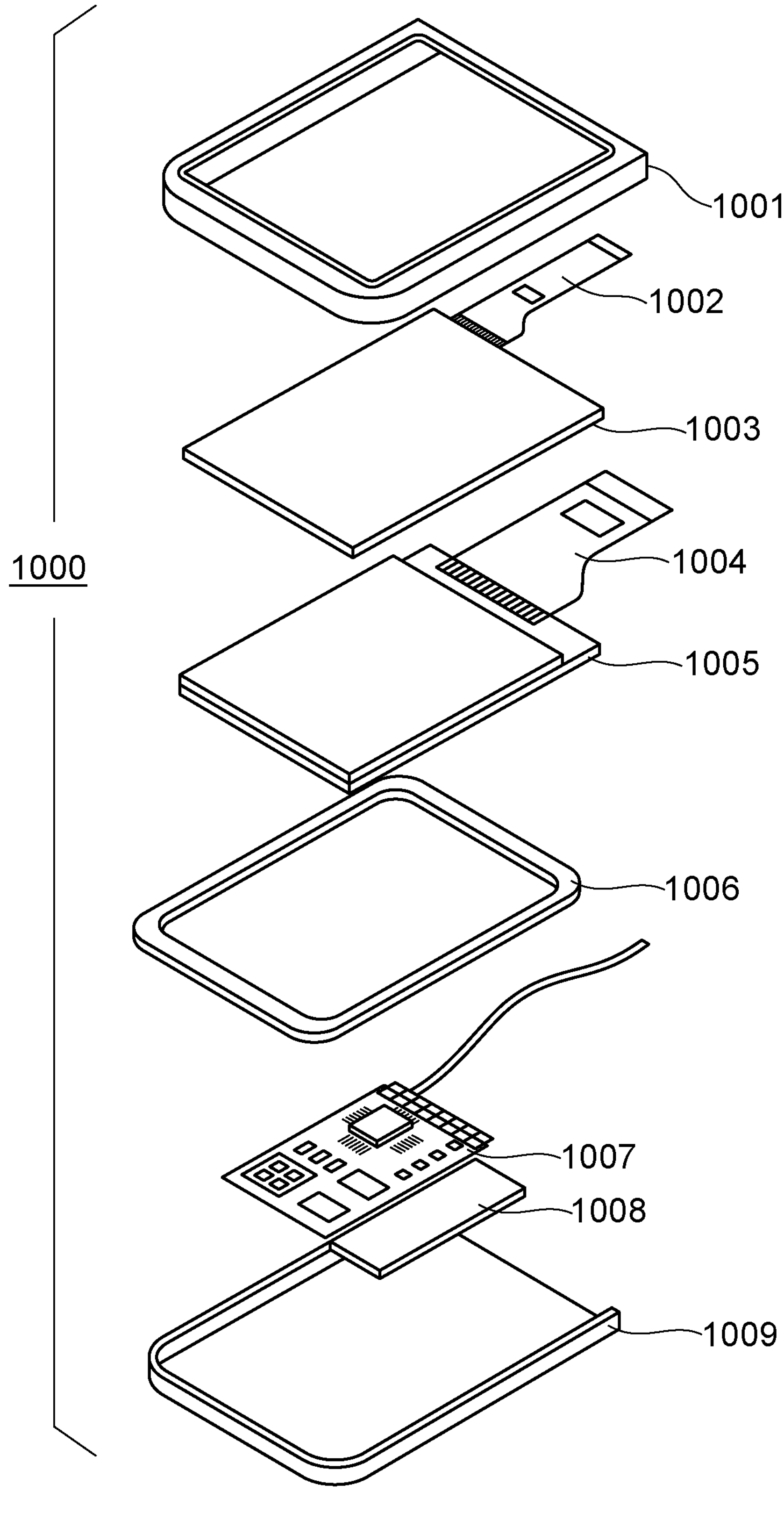

FIG. 4A

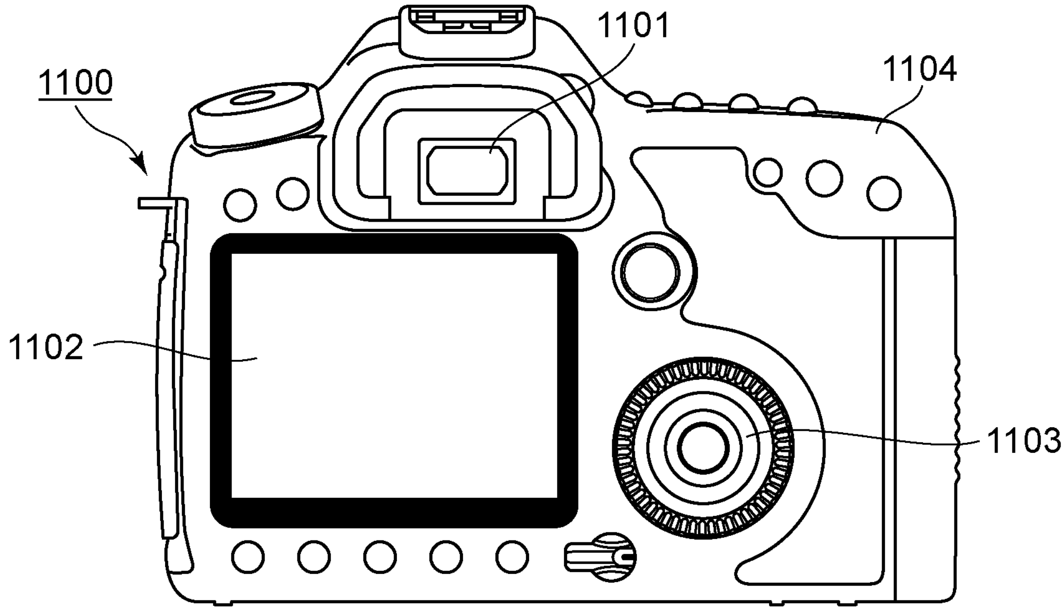


FIG. 4B

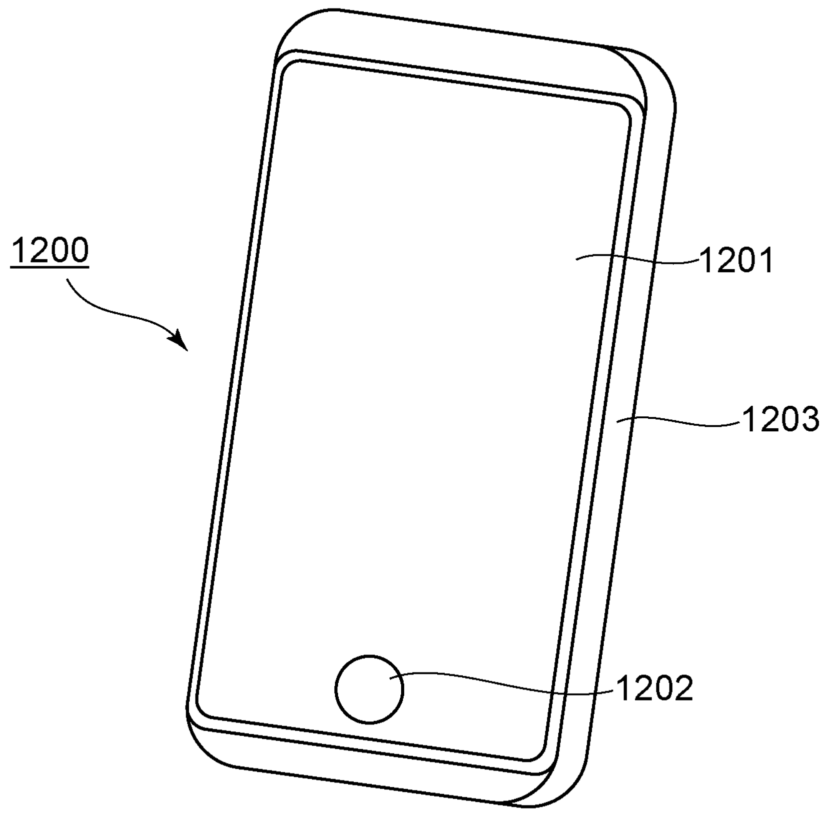

FIG. 5A

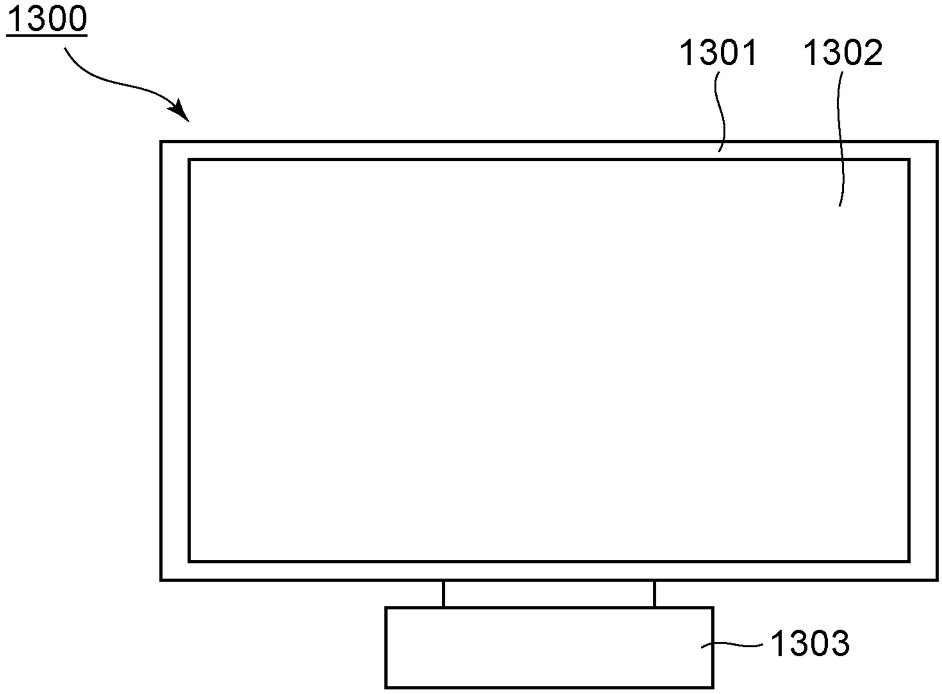


FIG. 5B

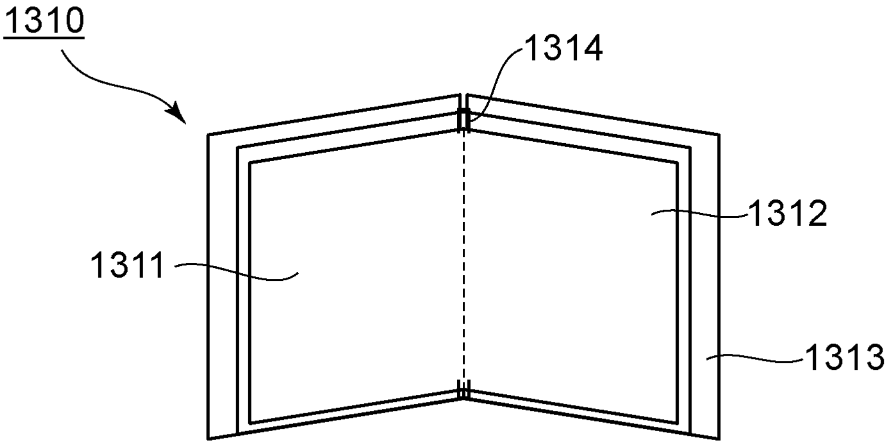

FIG. 6A

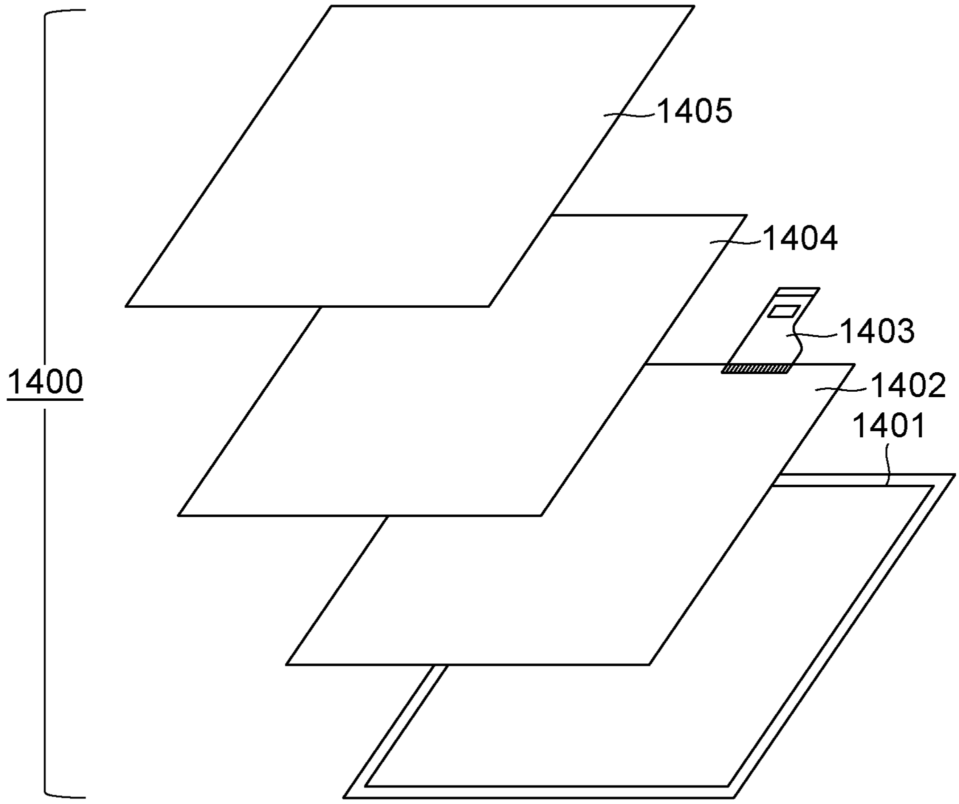


FIG. 6B

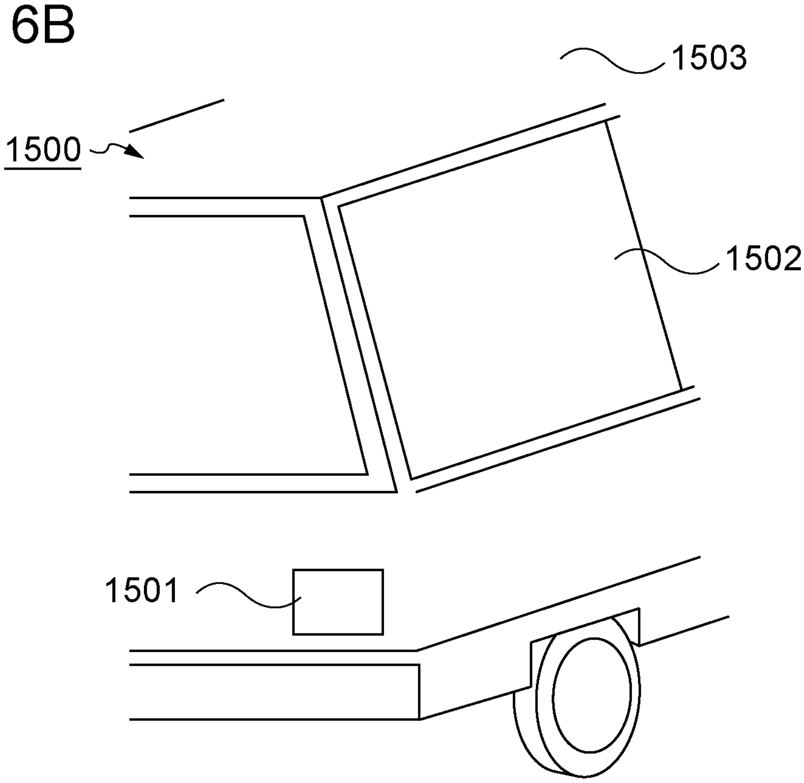

**INTERNATIONAL SEARCH REPORT**

International application No. PCT/JP2020/038736

A. CLASSIFICATION OF SUBJECT MATTER

C07C 22/08(2006.01)i; C07C 25/22(2006.01)i; C07C 255/51(2006.01)i; C07C 255/52(2006.01)i; C07C 255/54(2006.01)i; C09K 11/06(2006.01)i; G02B 5/20(2006.01)i

FI: C07C25/22 CSP; C07C255/51; C07C255/54; C07C255/52; C07C22/08; G02B5/20 101; C09K11/06 610

According to International Patent Classification (IPC) or to both national classification and IPC

B. FIELDS SEARCHED

Minimum documentation searched (classification system followed by classification symbols)

C07C22/00; C07C25/00; C07C255/00; C09K11/00; G02B5/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X | JP 2013-49663 A (CANON INC.) 14 March 2013 (2013-03-14) claims 1-13, page 17, compound B7 | 1-18 |
| X | JP 2009-302470 A (MITSUI CHEMICALS, INC.) 24 December 2009 (2009-12-24) claim 1, pp. 14-16, compounds 20, 23, 30-31 | 1-7 |
| X | JP 2005-89674 A (TOYO INK MANUFACTURING CO., LTD.) 07 April 2005 (2005-04-07) page 27, formula 17, compounds, page 33, compounds 131-134 | 1-18 |
| X | JP 2003-238516 A (SEKIYU SANGYO KASSEIKA CENTER) 27 August 2003 (2003-08-27) claims 1-10, page 14, compounds 10-1 to 10-6 | 1-18 |
| A | JP 2018-85499 A (CANON INC.) 31 May 2018 (2018-05-31) claims 1-16 | 1-18 |
| A | JP 2017-143133 A (CANON INC.) 17 August 2017 (2017-08-17) claims 1-18 | 1-18 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 09 December 2020 (09.12.2020) | 22 December 2020 (22.12.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application no.
PCT/JP2020/038736

| Patent Documents referred in the Report | Publication Date | Patent Family | Publication Date |
|---|---|---|---|
| JP 2013-49663 A | 14 Mar. 2013 | US 2013/0033416 A1 claims 1-13, page 26, compound B7<br>CN 102992942 A | |
| JP 2009-302470 A | 24 Dec. 2009 | (Family: none) | |
| JP 2005-89674 A | 07 Apr. 2005 | (Family: none) | |
| JP 2003-238516 A | 27 Aug. 2003 | (Family: none) | |
| JP 2018-85499 A | 31 May 2018 | US 2019/0267411 A1 claims 1-15<br>WO 2018/088325 A1<br>EP 3540804 A<br>CN 109937489 A | |
| JP 2017-143133 A | 17 Aug. 2017 | US 2018/0342559 A1 claims 1-19<br>WO 2017/138363 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 11040360 A
- JP 2019200318 A


### Non-patent literature cited in the description


- **WEINHOLD, F. ; LANDIS, C. R.** Natural bond orbitals and extensions of localized bonding concepts. *Chem. Educ. Res. Pract. Eur.,* 2001, vol. 2, 91-104